# FASCICULE DE BREVET EUROPEEN

(11) **EP 2 238 110 B1**
(45) Date de publication et mention de la délivrance du brevet: **26.10.2011**
(21) Numéro de dépôt: 09721603.0
(22) Date de dépôt: 05.02.2009
(51) Int. Cl.: C07D 213/60, C07D 401/04, A61K 31/435, A61P 9/00

(54) **DÉRIVÉS DE 5.6-BISARYL-2-PYR1DINE-CARBOXAMIDE, LEUR PRÉPARATIO LEUR APPLICATION EN THÉRAPEUTIQUE COMME ANTAGONISTES DES RECEPTEURS A L'UROTENSINE II**
5,6-BISARYL-2-PYRIDIN-CARBOXAMID-DERIVATE SOWIE IHRE HERSTELLUNG UND ANWENDUNG IN THERAPEUTIKA ALS UROTENSIN-II-REZEPTOR-ANTAGONISTEN
5.6-BISARYL-2-PYRIDINE-CARBOXAMIDE DERIVATIVES, PREPARATION THEREOF AND THERAPEUTIC APPLICATION THEREOF AS ANTAGONISTS FOR UROTENSINE II RECEPTORS

(30) Priorité: 07.02.2008 FR 0800651
(43) Date de publication de la demande: 13.10.2010
(73) Titulaire: SANOFI, 75013 Paris (FR)
(72) Inventeur: ALTENBURGER Jean-Michel, F-75013 PARIS (FR); FOSSEY Valérie, F-75013 PARIS (FR); GALTIER Daniel, F-75013 PARIS (FR); PETIT Frédéric, F-75013 PARIS - FRANCE (FR)
(74) Mandataire: Fischer, Hans-Jürgen
(86) Numéro de dépôt international: PCT/FR2009/000128
(87) Numéro de publication internationale: WO 2009/115665

(56) Documents cités:
- WO-A-03/051850
- WO-A-2008/020124
- WO-A2-2004/073634
- WO-A2-2004/078114
- FR-A- 2 856 684

## Description

La présente invention a pour objet des 5,6-bisaryl-2-pyridine-carboxamide, leur préparation et leur application en thérapeutique en tant qu'antagonistes des récepteurs à l'Urotensine II.

L'Urotensine II est un peptide cyclique constitué de 11 acides aminés et considéré comme étant l'un des plus puissants agents vasoconstricteurs connus à ce jour (Ames et al., 1999, Nature 401,282-286). Son activité biologique est médiée par l'activation d'un récepteur à 7 domaines transmembranaires couplé aux protéines G, le GPR14, renommé **UT (Urotensin II Receptor) par l'International Union of Basic and Clinical Pharmacology (IUPHAR)**. L'activation du récepteur de l'Urotensine II engendre une mobilisation du calcium intracellulaire. L'Urotensine II et son récepteur sont fortement exprimés au niveau du système cardiovasculaire, mais aussi au niveau rénal, cérébrale et dans le système endocrinien (Richards and Charles, 2004, Peptides 25,1795-1802). Sur vaisseaux isolés, l'Urotensine II humaine provoque une vasoconstriction dont l'intensité varie en fonction du territoire et de l'espèce concernés (Douglas et al., 2000, Br. J. Phamacol. 131,1262-1274). L'administration d'Urotensine II chez le primate anesthésié induit une augmentation des résistances vasculaires périphériques et une dégradation de la contractilité et du débit cardiaque, pouvant conduire à fortes doses à un collapsus cardiovasculaire et en définitive à la mort de l'animal (Ames et al., 1999, Nature 401,282-286). Par ailleurs l'Urotensine II stimule la prolifération des cellules musculaires lisses vasculaires et agit en synergie avec l'activité mitogénique de la sérotonine et des LDL **(Low Density Lipoproteins)** oxydés (Watanabe et al., 2001, Circulation 104 ;16-18). Sur les cardiomyocytes en culture, l'Urotensine II induit une hypertrophie cellulaire et une augmentation de la synthèse de matrice extracellulaire (Tzanidis A., et al, 2003, Circ. Res. 93, 246-253).

Les taux plasmatiques et urinaires d'Urotensine II ont été rapportés pour être augmentés dans un certain nombre de pathologies cardiovasculaires, rénales et métaboliques chez l'homme. Ces pathologies incluent l'hypertension artérielle, l'insuffisance cardiaque, l'insuffisance rénale, le diabète et la cirrhose hépatique (Richards and Charles, 2004, Peptides 25,1795-1802 ; Doggrell, 2004, Expert Opin Investig Drugs 13, 479-487).

Des effets centraux de l'Urotensine II ont également été décrits (Matsumoto Y., et al, Neurosci. Lett., 2004, 358, 99).

Enfin, il a été montré que certaines lignées de cellules tumorales surexpriment le récepteur de l'Urotensine II (Takahashi K., et al, Peptides, 2003, 24, 301).

Les antagonistes des récepteurs de l'Urotensine II peuvent être utiles pour le traitement de l'insuffisance cardiaque congestive, l'ischémie cardiaque, l'infarctus du myocarde, l'hypertrophie et la fibrose cardiaque, les maladies coronaires et l'athérosclérose, l'hypertension artérielle systémique et pulmonaire, l'hypertension portale et la fibrose hépatique, la resténose post-angioplastie, les insuffisances rénales aïgue et chronique d'origine diabétique et/ou hypertensive, le diabète, l'inflammation vasculaire, et les anévrismes. Par ailleurs les antagonistes des récepteurs de l'Urotensine II peuvent être utiles pour le traitement des désordres du système nerveux central, incluant les maladies neurodégénératives, les accidents vasculaires cérébraux, le stress, l'anxiété, l'agressivité, la dépression, la schizophrénie mais aussi les vomissements et les maladies du sommeil. Enfin, les antagonistes des récepteurs de l'Urotensine II peuvent également être utiles pour le traitement de certains cancers.

Les composés selon la présente invention répondent à la formule (I) : 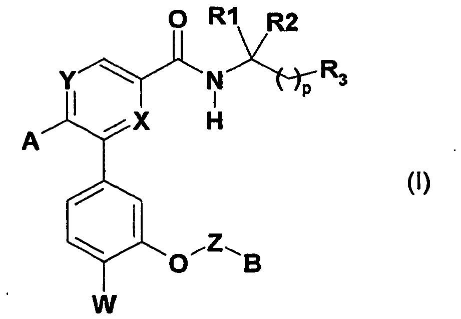 dans laquelle :
X et Y représentent indépendamment l'un de l'autre un atome d'azote ou un chaînon -CR4-, où R4 représente un atome d'hydrogène ou un groupe (C1-C4) alkyle ou alcoxy ;

A représente un groupe aryle ou hétéroaryle, lesdits groupes aryle ou hétéroaryle étant éventuellement substitués par un ou plusieurs groupes choisis parmi un atome d'halogène, un groupe hydroxy, (C1-C4) alkyle, (C3-C5)cycloalkyle, (C1-C4) alcoxy éventuellement substitué par un groupe (C1-C4)alcoxy, halogénoalkyle, halogénoalcoxy, un groupe nitrile ;
W représente un atome d'halogène ou un groupe halogénoalkyle ;
Z représente un groupe (C1-C4)alkylène éventuellement substitué par un ou plusieurs groupes choisis parmi un atome d'halogène et les groupes (C1-C4)alkyle, hydroxy et (C1-C4)alcoxy ;
B représente un groupe -NR4R5, où R4 et R5 représentent indépendamment l'un de l'autre un groupe (C1-C4)alkyle ;
R1 et R2 représentent :
- soit, R1 représente un atome d'hydrogène et R2 représente un groupe (C1-C4)alkyle,
- soit R1 et R2 forment ensemble, avec l'atome de carbone auquel ils sont rattachés, un système mono ou polycyclique choisi parmi : un groupe (C3-C8)cycloalkyle, un groupe bicyclique ponté ou un groupe tetracyclique ponté, ledit système pouvant être substitué par un ou plusieurs groupes hydroxy ;
R3 représente :
- soit un groupe C(O)R5 avec R5 représente un groupe (C1-C4)alcoxy éventuellement substitué par un groupe (C1-C4)alcoxy ou un groupe NR6R7 avec R6 et R7 indépendamment l'un de l'autre représentent un atome d'hydrogène, un groupe (C1-C4)alkyle, (C3-C5)cycloalkyle, (C1-C4)alkylesulfonyle, halogénoalkyle,
- soit un groupe CH₂XR8 avec :
   - X représente un atome d'oxygène et R8 représente un atome d'hydrogène ou un groupe (C1-C4)alkyle,
   - ou X représente un groupe NH et R8 représente un groupe (C1-C4)alkylecarbonyle, (C1-C4)alkylecarboxyle ou (C1-C4)alkylesulfonyle,
- soit un groupe nitrile (CN) ;
p représente un nombre entier égal à 0 ou 1.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou salifiés par des acides ou des bases, notamment des acides ou des bases pharmaceutiquement acceptables. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules d'eau ou de solvant. De tels hydrates et solvats font également partie de l'invention.

Parmi les composés décrits dans la présente invention, on peut citer un premier groupe de composés répondant à la formule (I) dans laquelle :
X et Y représentent indépendamment l'un de l'autre un atome d'azote ou un chaînon -CR4-, où R4 représente un atome d'hydrogène ;

A représente un groupe aryle ou hétéroaryle, les dits groupes aryle ou hétéroaryle étant éventuellement substitués par un ou plusieurs groupes choisis parmi un atome d'halogène, un groupe hydroxy, (C1-C4) alkyle, (C3-C5)cycloalkyle, (C1-C4) alcoxy éventuellement substitué par un groupe (C1-C4)alcoxy, halogénoalkyle, halogénoalcoxy ;
W représente un atome d'halogène ;
Z représente un groupe (C1-C4)alkylène ;
B représente un groupe -NR4R5, où R4 et R5 représentent indépendamment l'un de l'autre un groupe (C1-C4)alkyle,
R1 et R2 représentent :
- soit, R1 représente un atome d'hydrogène et R2 représente un groupe (C1-C4)alkyle,
- soit R1 et R2 forment ensemble, avec l'atome de carbone auquel ils sont rattachés, un système mono ou polycyclique choisi parmi : un groupe (C3-C8)cycloalkyle ou un groupe tetracyclique ponté, ledit système pouvant être substitué par un ou plusieurs groupes hydroxy ;
R3 représente :
- soit un groupe C(O)R5 avec R5 représente un groupe (C1-C4)alcoxy éventuellement substitué par un groupe (C1-C4)alcoxy ou un groupe NR6R7 avec R6 et R7 indépendamment l'un de l'autre représentent un atome d'hydrogène, un groupe (C1-C4)alkyle, (C3-C5)cycloalkyle, (C1-C4)alkylesulfonyle, halogénoalkyle,
- soit un groupe CH₂XR8 avec X représente un atome d'oxygène et R8 représente un atome d'hydrogène ou un groupe (C1-C4)alkyle,
- soit un groupe nitrile (CN) ;
p représente un nombre entier égal à 0 ou 1.

Parmi les composés de formule (I) objets de l'invention, on peut citer un deuxième groupe de composés qui se définit comme suit :
X et Y représentent indépendamment l'un de l'autre un atome d'azote ou un chaînon -CR4-, où R4 représente un atome d'hydrogène ;
   et/ou
A représente un groupe aryle ou hétéroaryle, les dits groupes aryle ou hétéroaryle étant éventuellement substitués par un ou plusieurs groupes choisis parmi un atome d'halogène, un groupe hydroxy, (C1-C4) alkyle, (C3-C5)cycloalkyle, (C1-C4) alcoxy éventuellement substitué par un groupe (C1-C4) alcoxy, halogénoalkyle, halogénoalcoxy ;
et/ou
W représente un atome d'halogène ;
et/ou
Z représente un groupe (C1-C4)alkylène ;
et/ou
B représente un groupe -NR4R5, où R4 et R5 représentent indépendamment l'un de l'autre un groupe (C1-C4)alkyle,
et/ou
R1 et R2 représentent :
   - soit, R1 représente un atome d'hydrogène et R2 représente un groupe (C1-C4)alkyle,
   - soit R1 et R2 forment ensemble, avec l'atome de carbone auquel ils sont rattachés, un système mono ou polycyclique choisi parmi : un groupe (C3-C8)cycloalkyle ou un groupe tetracyclique ponté, ledit système pouvant être substitué par un ou plusieurs groupes hydroxy ;
et/ou
R3 représente :
   - soit un groupe C(O)R5 avec R5 représente un groupe (C1-C4)alcoxy éventuellement substitué par un groupe (C1-C4)alcoxy ou un groupe NR6R7 avec R6 et R7 indépendamment l'un de l'autre représentent un atome d'hydrogène, un groupe (C1-C4)alkyle, (C3-C5)cycloalkyle, (C1-C4)alkylesulfonyle, halogénoalkyle,
   - soit un groupe -CH₂XR8 avec X représente un atome d'oxygène et R8 représente un atome d'hydrogène ou un groupe (C1-C4)alkyle,
et/ou
p représente un nombre entier égal à 0 ou 1.

Parmi les composés de formule (I) objets de l'invention, on peut citer un troisième groupe de composés qui se définit comme suit :
X et Y représentent indépendamment l'un de l'autre un atome d'azote ou un chaînon -CR4-, où R4 représente un atome d'hydrogène ;
et/ou
A représente un groupe aryle ou hétéroaryle, les dits groupes aryle ou hétéroaryle étant éventuellement substitués par un ou plusieurs groupes choisis parmi un atome d'halogène, un groupe hydroxy, (C1-C4) alkyle, (C3-C5)cycloalkyle, (C1-C4) alcoxy éventuellement substitué par un groupe (C1-C4) alcoxy, halogénoalkyle, halogénoalcoxy ;
et/ou
W représente un atome d'halogène;
et/ou
Z représente un groupe (C1-C4)alkylène ;
et/ou
B représente un groupe -NR4R5, où R4 et R5 représentent indépendamment l'un de l'autre un groupe (C1-C4)alkyle,
et/ou
R1 et R2 représentent :
   - soit, R1 représente un atome d'hydrogène et R2 représente un groupe (C1-C4)alkyle,
   - soit R1 et R2 forment ensemble, avec l'atome de carbone auquel ils sont rattachés, un système mono ou polycyclique choisi parmi : un groupe (C3-C8)cycloalkyle ou un groupe tetracyclique ponté, ledit système pouvant être substitué par un ou plusieurs groupes hydroxy ;
et/ou
R3 représente :
   - soit un groupe C(O)R5 avec R5 représente un groupe (C1-C4)alcoxy éventuellement substitué par un groupe (C1-C4)alcoxy ou un groupe NR6R7 avec R6 et R7 indépendamment l'un de l'autre représentent un atome d'hydrogène, un groupe (C1-C4)alkyle, (C3-C5)cycloalkyle, (C1-C4)alkylesulfonyle, halogénoalkyle,
   - soit un groupe CH2XR8 avec X représente un atome d'oxygène et R8 représente un atome d'hydrogène ou un groupe (C1-C4)alkyle,
   - soit un groupe nitrile (CN) ;
et/ou
p représente un nombre entier égal à 0 ou 1.

Parmi les composés de formule (I) objets de l'invention, on peut citer un quatrième groupe de composés pour lesquels X et Y représentent indépendamment l'un de l'autre un atome d'azote ou un chaînon -CR4-, où R4 représente un atome d'hydrogène.

Parmi les composés de formule (I) objets de l'invention, on peut citer un cinquième groupe de composés pour lesquels A représente un groupe aryle ou hétéroaryle, les dits groupes aryle ou hétéroaryle étant éventuellement substitués par un ou plusieurs groupes choisis parmi un atome d'halogène, un groupe hydroxy, (C1-C4) alkyle, (C3-C5)cycloalkyle, (C1-C4) alcoxy éventuellement substitué par un groupe (C1-C4) alcoxy, halogénoalkyle, halogénoalcoxy.

Les groupes aryles et hétéroaryles du cinquième groupe peuvent être choisis parmi un groupe phényle, pipéronyle, pyridinyle ou pyrazolyle éventuellement relié au noyau central comportant X et Y par un atome d'azote.

Parmi les composés de formule (I) objets de l'invention, on peut citer un sixième groupe de composés pour lesquels W représente un atome d'halogène.

Parmi les composés de formule (I) objets de l'invention, on peut citer un septième groupe de composés pour lesquels Z représente un groupe (C1-C4)alkylène.

Parmi les composés de formule (I) objets de l'invention, on peut citer un huitième groupe de composés pour lesquels B représente un groupe -NR4R5, où R4 et R5 représentent indépendamment l'un de l'autre un groupe (C1-C4)alkyle.

Parmi les composés de formule (I) objets de l'invention, on peut citer un neuvième groupe de composés pour lesquels R1 représente un atome d'hydrogène et R2 représente un groupe (C1-C4)alkyle.

Parmi les composés de formule (I) objets de l'invention, on peut citer un dixième groupe de composés pour lesquels R1 et R2 forment ensemble, avec l'atome de carbone auquel ils sont rattachés, un système mono ou polycyclique choisi parmi : un groupe (C3-C8)cycloalkyle ou groupe tetracyclique ponté, ledit système pouvant être substitué par un ou plusieurs groupes hydroxy.

Ledit groupe tetracyclique ponté du dixième groupe peut être un groupe adamantyle.

Parmi les composés de formule (I) objets de l'invention, on peut citer un onzième groupe de composés pour lesquels R3 représente un groupe C(O)R5 avec R5 représente un groupe (C1-C4)alcoxy éventuellement substitué par un groupe (C1-C4)alcoxy ou un groupe NR6R7 avec R6 et R7 indépendamment l'un de l'autre représentent un atome d'hydrogène, un groupe (C1-C4)alkyle, (C3-C5)cycloalkyle, (C1-C4)alkylesulfonyle, halogénoalkyle.

Parmi les composés de formule (I) objets de l'invention, on peut citer un douzième groupe de composés pour lesquels R3 représente un groupe -CH₂XR8 avec X représente un atome d'oxygène et R8 représente un atome d'hydrogène ou un groupe (C1-C4)alkyle.

Parmi les composés de formule (I) objets de l'invention, on peut citer un treizième groupe de composés pour lesquels p représente un nombre entier égal à 0.

Parmi les composés de formule (I) objets de l'invention, on peut citer un quatorzième groupe de composés pour lesquels p représente un nombre entier égal à 1.

Tous les groupes ou sous-groupes peuvent être utilisés indépendamment les uns des autres en combinaison pour obtenir des composés selon l'invention.

Parmi les combinaisons de groupes correspondant à des composés objets de l'invention, on peut citer une première combinaison correspondant à des composés pour lesquels :
X représente un atome d'azote et Y représente un atome d'azote ou un chaînon - CR4-, où R4 représente un atome d'hydrogène ;

A représente un groupe aryle étant éventuellement substitué par un ou plusieurs groupes choisis parmi un atome d'halogène, (C1-C4)alkyle, (C3-C5)cycloalkyle, (C1-C4)alcoxy, halogénoalcoxy, ou représente un groupe hétéroaryle étant éventuellement substitué par un ou plusieurs groupes choisis parmi un atome d'halogène, (C1-C4)alkyle;
W représente un atome d'halogène,
Z représente un groupe (C1-C4)alkylène ;
B représente un groupe -NR4R5, où R4 et R5 représentent indépendamment l'un de l'autre un groupe (C1-C4)alkyle,
R1 et R2 représentent :
   - soit, R1 représente un atome d'hydrogène et R2 représente un groupe (C1-C4)alkyle,
   - soit R1 et R2 forment ensemble, avec l'atome de carbone auquel ils sont rattachés, un groupe (C3-C8)cycloalkyle éventuellement susbtitué par un ou plusieurs groupes hydroxy ou un groupe adamantyle ;
R3 représente :
   - soit un groupe C(O)R5 avec R5 représente un groupe (C1-C4)alcoxy éventuellement substitué par un groupe (C1-C4)alcoxy ou un groupe NR6R7 avec R6 représente un atome d'hydrogène et R7 représente un groupe (C1-C4)alkyle, (C3-C5)cycloalkyle, (C1-C4)alkylesulfonyle, halogénoalkyle,
   - soit un groupe CH₂XR8 avec X représente un atome d'oxygène et R8 représente un atome d'hydrogène ou un groupe (C1-C4)alkyle ;
p représente un nombre entier égal à 0 ou 1.

Parmi les combinaisons de groupes correspondant à des composés objets de l'invention, on peut citer une deuxième combinaison correspondant à des composés pour lesquels :
X représente un atome d'azote et Y représente un atome d'azote ou un chaînon - CR4-, où R4 représente un atome d'hydrogène ;

A représente un groupe aryle éventuellement substitué par un ou plusieurs groupes choisis parmi un atome d'halogène, un groupe (C1-C4) alkyle ou un groupe (C1-C4) alcoxy ou représente un groupe hétéroaryle éventuellement substitué par un ou plusieurs atomes d'halogène ou un ou plusieurs groupes (C1-C4) alkyle,
W représente un atome d'halogène,
Z représente un groupe (C1-C4)alkyléne ;
B représente un groupe -NR4R5, où R4 et R5 représentent indépendamment l'un de l'autre un groupe (C1-C4)alkyle,
R1 et R2 représentent :
   - soit, R1 représente un atome d'hydrogène et R2 représente un groupe (C1-C4)alkyle,
   - soit R1 et R2 forment ensemble, avec l'atome de carbone auquel ils sont rattachés, un groupe (C3-C8)cycloalkyle ou un groupe adamantyle ;
R3 représente :
   - soit un groupe C(O)R5 avec R5 représente un groupe (C1-C4)alcoxy éventuellement substitué par un groupe (C1-C4)alcoxy ou un groupe NR6R7 avec R6 représente un atome d'hydrogène et R7 représente un atome d'hydrogène ou un groupe (C1-C4)alkyle, (C3-C5)cycloalkyle, (C1-C4)alkylesulfonyle,
   - soit un groupe -CH₂XR8 avec X représente un atome d'oxygène et R8 représente un atome d'hydrogène ;
p représente un nombre entier égal à 0 ou1.

Parmi les combinaisons de groupes correspondant à des composés objets de l'invention, on peut citer une troisième combinaison correspondant à des composés pour lesquels :
X représente un atome d'azote et Y représente un chaînon -CR4-, où R4 représente un atome d'hydrogène;

A représente un groupe aryle étant éventuellement substitué par un ou plusieurs groupes choisis parmi un atome d'halogène, (C1-C4)alkyle, (C1-C4)alcoxy ou représente un groupe hétéroaryle éventuellement substitué par un ou plusieurs atomes d'halogène;
W représente un atome d'halogène,
Z représente un groupe (C1-C4)alkylène ;
B représente un groupe -NR4R5, où R4 et R5 représentent indépendamment l'un de l'autre un groupe (C1-C4)alkyle,
R1 et R2 représentent :
   - soit, R1 représente un atome d'hydrogène et R2 représente un groupe (C1-C4)alkyle, l'atome de carbone qui porte R1 et R2 étant de configuration absolue (S),
   - soit R1 et R2 forment ensemble, avec l'atome de carbone auquel ils sont rattachés, un groupe (C3-C8)cycloalkyle ;
R3 représente :
   - soit un groupe C(O)R5 avec R5 représente un groupe (C1-C4)alcoxy éventuellement substitué par un groupe (C1-C4)alcoxy ou un groupe NR6R7 avec R6 représente un atome d'hydrogène et R7 représente un atome d'hydrogène, un groupe (C1-C4)alkyle, (C3-C5)cycloalkyle, (C1-C4)alkylesulfonyle, halogénoalkyle,
   - soit un groupe -CH₂XR8 avec X représente un atome d'oxygène et R8 représente un atome d'hydrogène ou un groupe (C1-C4)alkyle ;
p représente un nombre entier égal à 1.

Dans le cadre de la présente invention, et sauf mention différente dans le texte, on entend par :
- un atome d'halogène: un atome de fluor, de chlore, de brome ou d'iode ;
- un groupe alkyle : un groupe aliphatique saturé linéaire, comprenant de 1 à 5 atomes de carbone ou quand la chaîne alkyle comprend au moins trois atomes de carbone pouvant être linéaire, ramifié ou partiellement cyclisé. A titre d'exemples, on peut citer les groupes méthyle, éthyle, propyle, isopropyle, butyle, isobutyle, tertbutyle, pentyle, méthylène-cyclopropyle ;
- un groupe alkylène : un groupe alkyle tel que défini ci-dessus, qui est divalent. A titre d'exemple, on peut citer un groupe méthylène, propylène, butylène, éthylène (-CH₂-CH₂-), ou 2-méthylpropylène ;
- un groupe cycloalkyle : un groupe cyclique saturé, qui comprend de 3 à 8 atomes de carbone et qui est cyclique. A titre d'exemples, on peut citer les groupes cyclopropyle, cyclopentyle, cyclohexyle ;
- un groupe polycyclique ponté : système polycyclique comprenant deux à quatre cycles saturés de 7 à 10 atomes de carbone dont des carbones adjacents sont reliés entre eux et appartiennent à au moins deux cycles en même temps, par exemple un groupe bicyclique ponté ou un groupe tetracyclique ponté tel que le groupe adamantyle ;
- un groupe aryle : un groupe aromatique monocyclique comprenant 5 ou 6 atomes de carbones, par exemple un groupe phényle, ce cycle pouvant être fusionné avec un groupe hétérocyclique partiellement saturé comprenant 5 ou 6 atomes dont un ou deux hétéroatomes tels qu'un atome d'oxygène, par exemple un groupe dioxolyle pour former un groupe pipéronyle ;
- un groupe hétéroaryle : un groupe aromatique cyclique comprenant 5 ou 6 atomes dont un ou plusieurs hétéroatomes tels qu'un atome d'azote. A titre d'exemple de groupes hétéroaryles, on peut citer un groupe pyridinyle, pyrazolyte ;
- un groupe cycloalkylène : un groupe cycloalkyle tel que défini ci-dessus qui est divalent ;
- un groupe halogénoalkyle : un groupe alkyle tel que défini ci-dessus, dont un ou plusieurs atomes d'hydrogène ont été substitués par un atome d'halogène. A titre d'exemple, on peut citer le groupe trifluorométhyle, difluorométhyle ;
- un groupe halogénoalcoxy : un groupe de formule -O-halogénoalkyle où le groupe halogénoalkyle est tel que défini ci-dessus. A titre d'exemple, on peut citer le groupe -O-CHF₂ ;
- un groupe alcoxy : un groupe de formule -O-alkyle où le groupe alkyle est tel que précédemment défini.

Parmi les composés objets de l'invention, on peut notamment citer les composés suivants :
1. 1-{[(3,5-dichloro-2'-{4-chloro-3-[3-(diméthylamino)propoxy]phényl}-2,3'-bipyridin-6'-yl)carbonyl]amino}cyclohexanecarboxylate de méthyle ;
2. 1-({[6-{4-chloro-3-[3-(diméthylamino)propoxy]phényl}-5-(4-hydroxy-2-méthylphényl)pyridin-2-yl]carbonyl}amino)cyclohéxanecarboxylate de méthyle ;
3. 1-({[6-{4-chloro-3-[3-(diméthylamino)propoxy]phényl}-5-(5-hydroxy-2-méthylphényl)pyridin-2-yl]carbonyl}amino)cyclohexanecarboxylate de méthyle ;
4. 1-({[6-{4-chloro-3-[3-(diméthylamino)propoxy]phényl}-5-(2-méthylphényl)pyridin-2-yl]carbonyl}amino)cyclohexanecarboxylate de méthyle ;
5. 1-{[(3-chloro-2'-{4-chloro-3-[3-(diméthylamino)propoxy]phényl}-5-méthyl-2,3'-bipyridin-6'-yl)carbonyl]amino}cyclohexanecarboxylate de méthyle ;
6. 1-{[(6-{4-chloro-3-[3-(diméthylamino)propoxy]phényl}-5-[5-(2-méthoxyéthoxy)-2-méthylphényl]pyridin-2-yl)carbonyl]amino}cyclohexanecarboxylate de méthyle ;
7. 1-({[6-{4-chloro-3-[3-(diméthylamino)propoxy]phényl}-5-(5-cyclopropyl-2-méthylphényl)pyridin-2-yl]carbonyl}amino)cyclohexanecarboxylate de méthyle ;
8. 1-({[6-{4-chloro-3-[3-(diméthylamino)propoxy]phényl}-5-(5-isopropoxy-2-méthylphényl)pyridin-2-yl]carbonyl}amino)cyclohexanecarboxylate de méthyle ;
9. 1-({[6-{4-chloro-3-[3-(diméthylamino)propoxy]phényl}-5-(2-chloro-5-propoxyphényl)pyridin-2-yl]carbonyl}amino)cyclohexanecarboxylate de méthyle ;
10. 1-({[6-{4-chloro-3-[3-(diméthylamino)propoxy]phényl}-5-(5-éthoxy-2-méthylphényl)pyridin-2-yl]carbonyl}amino)cyclohexanecarboxylate de méthyle ;
11. 1-({[6-{4-chloro-3-[3-(diméthylamino)propoxy]phényl}-5-(6-méthyl-1,3-benzodioxol-5-yl)pyridin-2-yl]carbonyl}amino)cyclohexanecarboxylate de méthyle ;
12. 1-{[(3-chloro-2'-{4-chloro-3-[3-(diméthylamino)propoxy]phényl}-5-fluoro-2,3'-bipyridin-6'-yl)carbonyl]amino}cyclohéxanecarboxylate de méthyle ;
13. 1-{[(2'-{4-chloro-3-[3-(diméthylamino)propoxy]phényl}-3,5-diméthyl-2,3'-bipyridin-6'-yl)carbonyl]amino}cyclohexanecarboxylate de méthyle ;
14. 1-({[6-{4-chloro-3-[3-(diméthylamino)propoxy]phényl}-5-(3-hydroxy-2-méthylphényl)pyridin-2-yl]carbonyl}amino)cyclohexanecarboxylate de méthyle ;
15. 1-{[(2-{4-chloro-3-[3-(diméthylamino)propoxy]phényl}2'-méthyl-3,3'-bipyridin-6-yl)carbonyl]amino}cyclohexanecarboxylate de méthyle ;
16. 1-{[(6-{4-chloro-3-[3-(diméthylamino)propoxy]phényl}-5-[5-(difluorométhoxy)-2-méthylphényl]pyridin-2-yl)carbonyl]amino}cyclohexanecarboxylate de méthyle ;
17. 1-{[(6-{4-chloro-3-[3-(diméthylamino)propoxy]phényl}-5-[2-(difluorométhyl)-5-méthylphényl]pyridin-2-yl)carbonyl]amino}cyclohexanecarboxylate de méthyle ;
18. 1-({[6-{4-chloro-3-[3-(diméthylamino)propoxy]phényl}-5-(3,5-diméthyl-1*H*-pyrazol-1-yl)pyridin-2-yl]carbonyl}amino)cyclohéxanecarboxylate de méthyle ;
19. 1-({[6-{4-chloro-3-[3-(diméthylamino)propoxy]phényl}-5-(3,5-diéthyl-1*H*-pyrazol-1-yl)pyridin-2-yl]carbonyl}amino)cyclohexanecarboxylate de méthyle ;
20. 1-({[6-{4-chloro-3-[3-(diméthylamino)propoxy]phényl}-5-(2,4-diméthylphényl)pyrazin-2-yl]carbonyl}amino)cyclohexanecarboxylate de méthyle ;
21. 1-({[6-{4-chloro-3-[3-(diméthylamino)propoxy]phényl}-5-(2-chloro-5-éthoxyphényl)pyrazin-2-yl]carbonyl}amino)cyclohexanecarboxylate de méthyle ;
22. (3*S*)-3-({[6-{4-chloro-3-[3-(diméthylamino)propoxy]phényl}-5-(2-chloro-5-méthylphényl)pyridin-2-yl]carbonyl}amino)-4-méthylpentanoate de méthyle ;
23. (3*S*)-3-({[6-{4-chloro-3-[3-(diméthylamino)propoxy]phényl}-5-(2,4-diméthylphényl)pyridin-2-yl]carbonyl}amino)-4,4-diméthylpentanoate de méthyle ;
24. (3*S*)-3-({[6-{4-chloro-3-[3-(diméthylamino)propoxy]phényl}-5-(2-chloro-5-éthoxyphényl)pyrazin-2-yl]carbonyl}amino)-4,4-diméthylpentanoate de méthyle ;
25. (3*S*)-3-{[(3-chloro-2'-{4-chloro-3-[3-(diméthylamino)propoxy]phényl}-5-fluoro-2,3'-bipyridin-6'-yl)carbonyl]amino}-4,4-diméthylpentanoate de méthyle ;
26. 2-({[6-{4-chloro-3-[3-(diméthylamino)propoxy]phényl}-5-(2-chlorophényl)pyridin-2-yl]carbonyl}amino)adamantane-2-carboxylate de méthyle ;
27. (3*S*)-3-({[6-{4-chloro-3-[3-(diméthylamino)propoxy]phényl}-5-(2-méthylphényl)pyridin-2-yl]carbonyl}amino)-4,4-diméthylpentanoate de 2-methoxyéthyle ;
28. (3*S*)-3-({[6-{4-chloro-3-[3-(diméthylamino)propoxy]phényl}-5-(2-méthylphényl)pyridin-2-yl]carbonyl}amino)-4,4-diméthylpentanoate de méthyle ;
29. (3*S*)-3-({[6-{4-chloro-3-[3-(diméthylamino)propoxy]phényl}-5-(2-méthylphényl)pyridin-2-yl]carbonyl}amino)-4,4-diméthylpentanoate d'éthyle ;
30. (3*S*)-3-({[6-{4-chloro-3-[3-(diméthylamino)propoxy]phényl}-5-(2-méthylphényl)pyridin-2-yl]carbonyl}amino)-4,4-diméthylpentanoate d'isopropyle ;
31. (3*S*)-3-({[6-{4-chloro-3-[3-(diméthylamino)propoxy]phényl}-5-(2-chlorophényl)pyridin-2-yl]carbonyl}amino)-4,4-diméthylpentanoate de méthyle ;
32. *N*-[(1*S*)-1-(2-amino-2-oxoéthyl)-2,2-diméthylpropyl]-6-{4-chloro-3-[3-(diméthylamino)propoxy]phényl}-5-(2-méthylphényl)pyridine-2-carboxamide ;
33. 6-{4-chloro-3-[3-(diméthylamino)propoxy]phényl}-*N*-{(1*S*)-2,2-diméthyl-1-[2-(méthylamino)-2-oxoéthyl]propyl}-5-(2-méthylphényl)pyridine-2-carboxamide
34. 6-{4-chloro-3-[3-(diméthylamino)propoxy]phényl}-*N*-[(1*S*)-2,2-diméthyl-1-{2-[(méthylsulfonyl)amino]-2-oxoéthyl}propyl]-5-(2-méthylphényl)pyridine-2-carboxamide ;
35. 6-{4-chloro-3-[3-(diméthylamino)propoxy]phényl}-*N*-[(1*S*)-1-{2-[(1,1,1-trifluoroéthyl)amino]-2-oxoéthyl}-2,2-diméthylpropyl]-5-(2-méthylphényl)pyridine-2-carboxamide ;
36. 6-{4-chloro-3-[3-(diméthylamino)propoxy]phényl}-*N*-[(1*S*)-1-{2-[cyclopropyl(méthyl)amino]-2-oxoéthyl}-2,2-diméthylpropyl]-5-(2-méthylphényl)pyridine-2-carboxamide ;
37. 6-{4-chloro-3-[3-(diméthylamino)propoxy]phényl}-*N*-[(1*S*)-1-(2-hydroxyéthyl)-2,2-diméthylpropyl]-5-(2-méthylphényl)pyridine-2-carboxamide ;
38. 6-{4-chloro-3-[3-(diméthylamino)propoxy]phényl}-*N*-[(1*S*)-1-(2-méthoxyéthyl)-2,2-diméthylpropyl]-5-(2-méthylphényl)pyridine-2-carboxamide ;
39. 6-{4-chloro-3-[3-(diméthylamino)propoxy]phényl}-*N*-[(2*S*)-1-cyano-3,3-diméthylbutan-2-yl]-5-(2-méthylphényl)pyridine-2-carboxamide ;
40. *cis*-1-{[(3,5-dichloro-2'-{4-chloro-3-[3-(diméthylamino)propoxy]phényl}-2,3'-bipyridin- 6'-yl)carbonyl]amino}-4-hydroxycyclohexanecarboxylate de méthyle ;
41. *cis*-1-({[6-{4-chloro-3-[3-(diméthylamino)propoxy]phényl}-5-(2,4-diméthylphényl)pyridin-2-yl]carbonyl}amino)-4-hydroxycyclohexanecarboxylate de méthyle ;
42. *cis*-1-({[6-{4-chloro-3-[3-(diméthylamino)propoxy]phényl}-5-(2-méthylphényl)pyridin-2-yl]carbonyl}amino)-4-hydroxycyclohexanecarboxylate de méthyle ;
43. *cis*-1-({[6-{4-chloro-3-[3-(diméthylamino)propoxy]phényl}-5-(5-éthoxy-2-méthylphényl)pyridin-2-yl]carbonyl}amino)-4-hydroxycyclohexanecarboxylate de méthyle ;
44. *cis*-1-{[(3-chloro-2'-{4-chloro-3-[3-(diméthylamino)propoxy]phényl}-5-méthyl-2,3'-bipyridin-6'-yl)carbonyl]amino}-4-hydroxycyclohexanecarboxylate de méthyle.

II est à noter que les composés ci-dessus ont été nommés en nomenclature IUPAC par l'intermédiaire du logiciel ACD/Name, version10.0, Advanced Chemistry Development, Inc.: Toronto ON, Canada, www.acdlabs.com, **2006**.

Dans ce qui suit, on entend par groupe protecteur (GP) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 3rd Edition (John Wiley & Sons, Inc., New York).

On entend par groupe partant (T), dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution nucléophile ou lors d'une réaction de couplage organométallique, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

Conformément à l'invention, on peut préparer les composés de formule (I) selon le procédé qui suit, illustré dans le schéma de synthèse N°1.

Le couplage peptidique effectué entre l'acide (II) et des amines de formule (III) en présence d'un agent de couplage tel que le tétrafluoroborate de N-{(1H-benzotriazol-1-yloxy) (diméthylamino)méthylidène}-N-méthylméthanaminium (TBTU) ou le chlorhydrate de N-[3-(diméthylamino)propyl-N'-éthylcarbodiimide (EDC.HCl) avec ou sans N-hydroxysuccinimide (HONSu) et d'une base organique telle que la N,N-diisopropyléthylamine (DIEA) et dans un solvant polaire aprotique tel que l'acétonitrile ou le DMF permet d'obtenir les composés de formules (I) conformes à l'invention (étape i). Les amines de formule (III) étant synthétisées à partir de méthodes connues de l'homme de métier.

Dans le cas où R3 = C(O)R5 avec R5 représente un groupe (C1-C4)alcoxy, on peut également saponifier les composés de formule (I) à l'aide d'une base minérale forte telle que l'hydroxyde de sodium dans un mélange binaire tel que eau/méthanol maintenu à T.A. ou chauffé à reflux pour conduire, après acidification, aux acides de formule (IV) (étape ii).

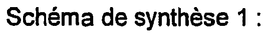

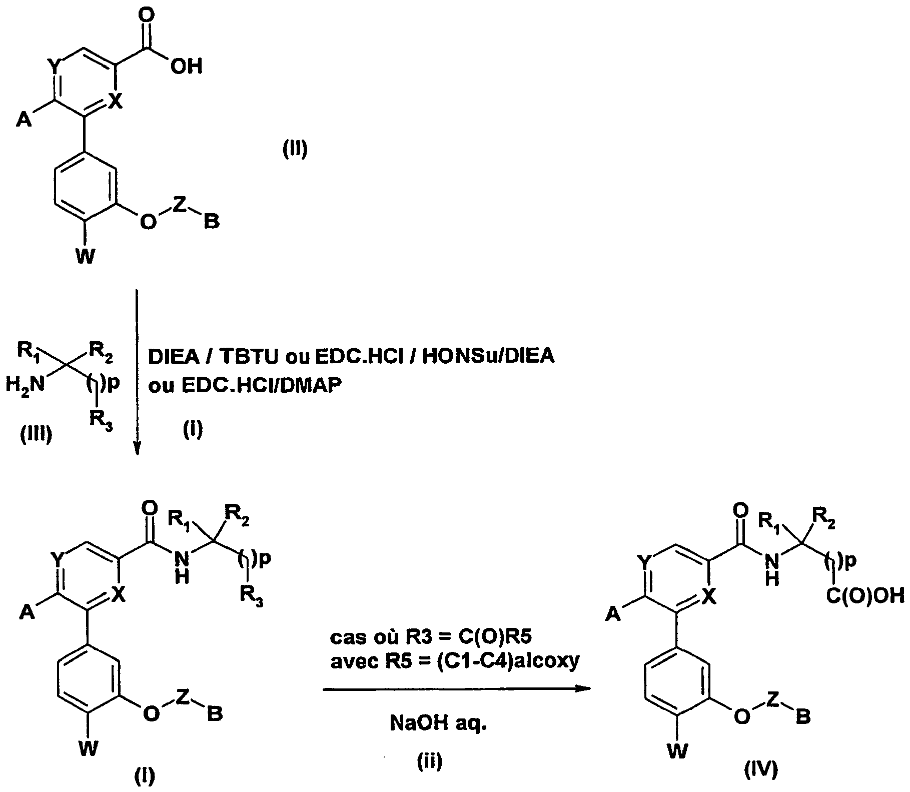

D'une autre façon, on peut préparer des composés de formule (la), qui correspondent à des composés de formule (I) pour lesquels R3 = C(O)R5, selon le procédé qui suit et illustré dans le schéma de synthèse N°2 :
La réaction entre l'acide (IV) et l'alcool ou l'amine R5H en présence de chlorure de thionyle (SOCl₂) ou d'un agent de couplage peptidique tel que le TBTU ou le système EDC.HCl/HONSu, d'une base organique telle que le DIEA et dans un solvant polaire aprotique tel que l'acétonitrile ou le DMF permet d'obtenir les composés de formule (la) conformes à l'invention (étape i).

Plus particulièrement, les composés de formule (Ib), qui correspondent à des composés de formule (I) pour lesquels R3 = -CH₂XR8, X étant un atome d'oxygène et R8 un atome d'hydrogène, peuvent être préparés par réduction de l'acide (IV) à l'aide d'un agent réducteur tel que le complexe borane diméthyl sulfure et dans un solvant aprotique tel que le THF (étape ii).

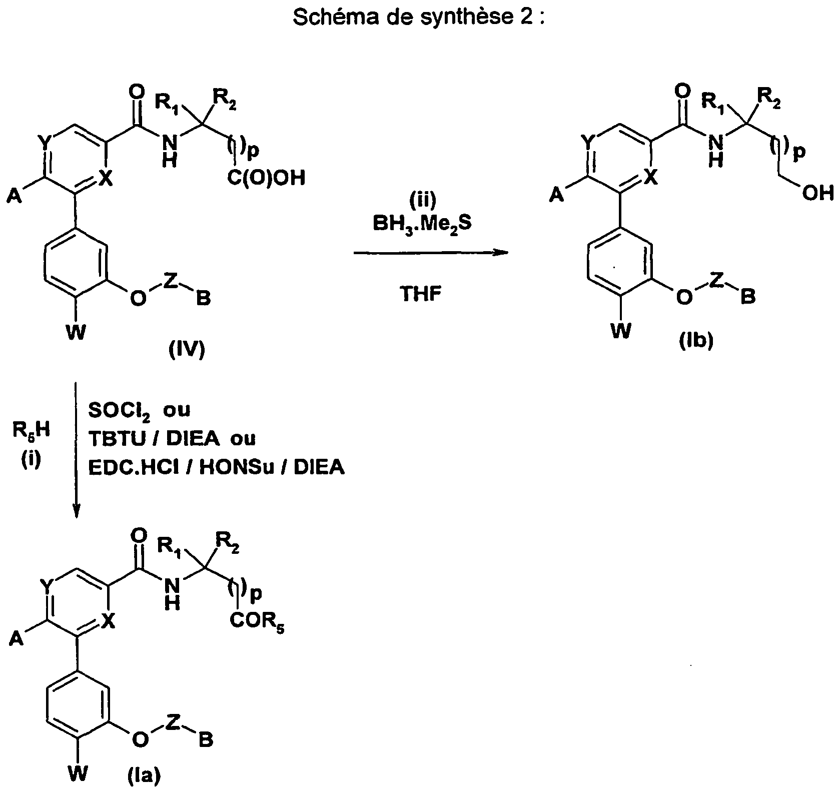

Les composés (II) et (IV) peuvent être préparés selon le procédé qui suit, illustré dans le schéma 3.

Quand, dans le composé de départ de formule (XIII), X représente un atome d'azote, Y représente un atome de carbone (c'est-à-dire un groupe de formule -CR4- telle que définie en rapport avec les composés de formule (I) selon l'invention), ou bien X représente un atome de carbone, Y représente un atome d'azote, ou encore X et Y représentent un atome d'azote, alors on peut effectuer dans une étape (i) une réaction de couplage de type SUZUKI catalysée par un dérivé de palladium (0) tel que le tétrakis(triphénylphosphine)palladium (0) [Pd(PPh₃)₄] ou de palladium (II) tel que le dichlorure de [1,1'-bis(cyclopentadiènyldiphénylphosphino)ferrocène] palladium (II) [PdCl₂(dppf)] entre le composé de formule (XIII) (où Q = OH ou Br et T = atome d'halogène, tel qu'un atome de brome ou d'iode) et un acide boronique de formule (XI), où GP représente un groupe benzyle éventuellement substitué par un ou plusieurs groupes alcoxy, en présence d'une base minérale faible telle que le phosphate de potassium ou le K₂CO₃ et dans un solvant polaire aprotique tel que le N,N-diméthylformamide (DMF) ou le 1,4-dioxanne à la température d'environ 95°C. Cette réaction permet de substituer régiosélectivement la fonction T par le noyau phénoxy (XI) pour conduire au composé (X).

La fonction OH du composé (X) est ensuite transformée en un groupe partant tel que trifluorométhanesulfonate (OTf), dans l'étape (ii), à l'aide d'anhydride trifluorométhanesulfonique en présence d'une base telle que la triéthylamine (TEA) et dans un solvant tel que le dichlorométhane (DCM) pour conduire au composé de formule (IX).

Le groupe trifluorométhanesulfonate ainsi obtenu permet dans l'étape (iii), grâce à sa réactivité, d'introduire le noyau A par une réaction de couplage organopalladiée de type :
- soit SUZUKI entre le composé (IX) et un acide ou un ester boronique de formules respectives A-B(OH)₂ ou 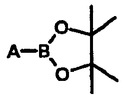 en présence d'une quantité catalytique de dérivé du palladium tel que le Pd(PPh₃)₄, en présence d'une base minérale faible telle que le phosphate de potassium et d'un solvant polaire aprotique tel que le DMF, à la température de 90°C ;
- soit STILLE entre le composé (IX) et un dérivé aryltributylstannane ou hétéroarylstannane ASnBu₃ en présence d'une quantité catalytique d'iodure de cuivre (Cul) et d'un dérivé de palladium (II) tel que le PdCl₂(dppf) et d'un solvant polaire aprotique tel que le 1,4-dioxanne à la température de 90°C ;

On obtient ainsi le composé (VIII).

Alternativement, on peut réaliser une inversion de réactivité en effectuant une séquence de deux réactions: BORYLATION / SUZUKI-MYAURA (étapes iv et v respectivement) à partir du dérivé (IX) et d'un halogénure d'aryle ou d'hétéroaryle A-T (T représente un atome d'halogène tel qu'un atome de brome ou d'iode). La réaction de SUZUKI-MYAURA est effectuée sur l'intermédiaire (XII) en présence d'une quantité catalytique d'un dérivé de palladium (o) tel que le Pd(PPh₃)₄, d'une base minérale faible telle que le carbonate de potassium et dans un mélange de solvants binaire tel que DME/eau à la température de 80°C environ. De plus, la fonction dioxoborolanyle du dérivé (XII) est obtenue à partir du groupe trifluorométhanesulfonyle du dérivé (IX), par réaction avec le 4,4,4',4',5,5,5',5'-octaméthyl-2,2'-bi-1,3,2-dioxaborolane en présence d'une quantité catalytique d'un dérivé du palladium (II) tel que le PdCl₂(dppf), d'une base faible telle que l'acétate de potassium et dans un solvant apolaire tel que le toluène à la température de 110°C environ.

On obtient ainsi le composé (VIII).

La déprotection de la fonction phénol du composé de formule (VIII) par le tribromure de bore à -78°C, l'acide trifluoroacétique (TFA) à T.A. ou le chlorure d'hydrogène à 0°C dans le DCM (étape (vi)) conduit au composé de formule (VII).

L'introduction du groupement Z-B dans l'étape (vii) peut être effectuée :
- soit par alkylation du composé (VII) avec un dérivé chloré CI-Z-B en présence d'une base minérale faible telle que le carbonate de césium et dans un solvant polaire aprotique tel que le DMF à une température comprise entre 80 et 100°C, telle que 90°C,
- soit par réaction de MITSUNOBU entre le composé (VII) et un alcool de formule HO-Z-B en présence de triphénylphosphine, d'azodicarboxylate de diisopropyle (DIAD), et d'une quantité catalytique de base organique faible telle que la TEA à 0°C dans un solvant aprotique tel que le tétrahydrofurane (THF).

Le composé de formule (VI) est ensuite saponifié dans l'étape (viii), à l'aide d'une base minérale forte telle que l'hydroxyde de potassium dans un mélange eau/méthanol maintenu à T.A. ou chauffé à reflux, pour conduire, après acidification avec un acide fort tel que l'acide chlorhydrique (HCl) 1N, au composé (II).

Dans le cadre de la présente invention par T.A., on entend une température comprise entre 20 et 25°C.

Une réaction de couplage peptidique (étape (ix)) entre le composé (II) et des amines de formule (V) en présence d'un agent de couplage tel que le carbonyldiimidazole (CDI), le tétrafluoroborate de N-[(1H-benzotriazol-1-yloxy)(diméthylamino)méthylidène]-N-méthylméthanaminium (TBTU) ou le système EDC.HCl/HONSu et d'une base organique telle que la DIEA et dans un solvant polaire aprotique tel que le DMF ou la N-méthylpyrrolidinone (NMP) ou l'acétonitrile à T.A. conduit aux composés de formule (IV). 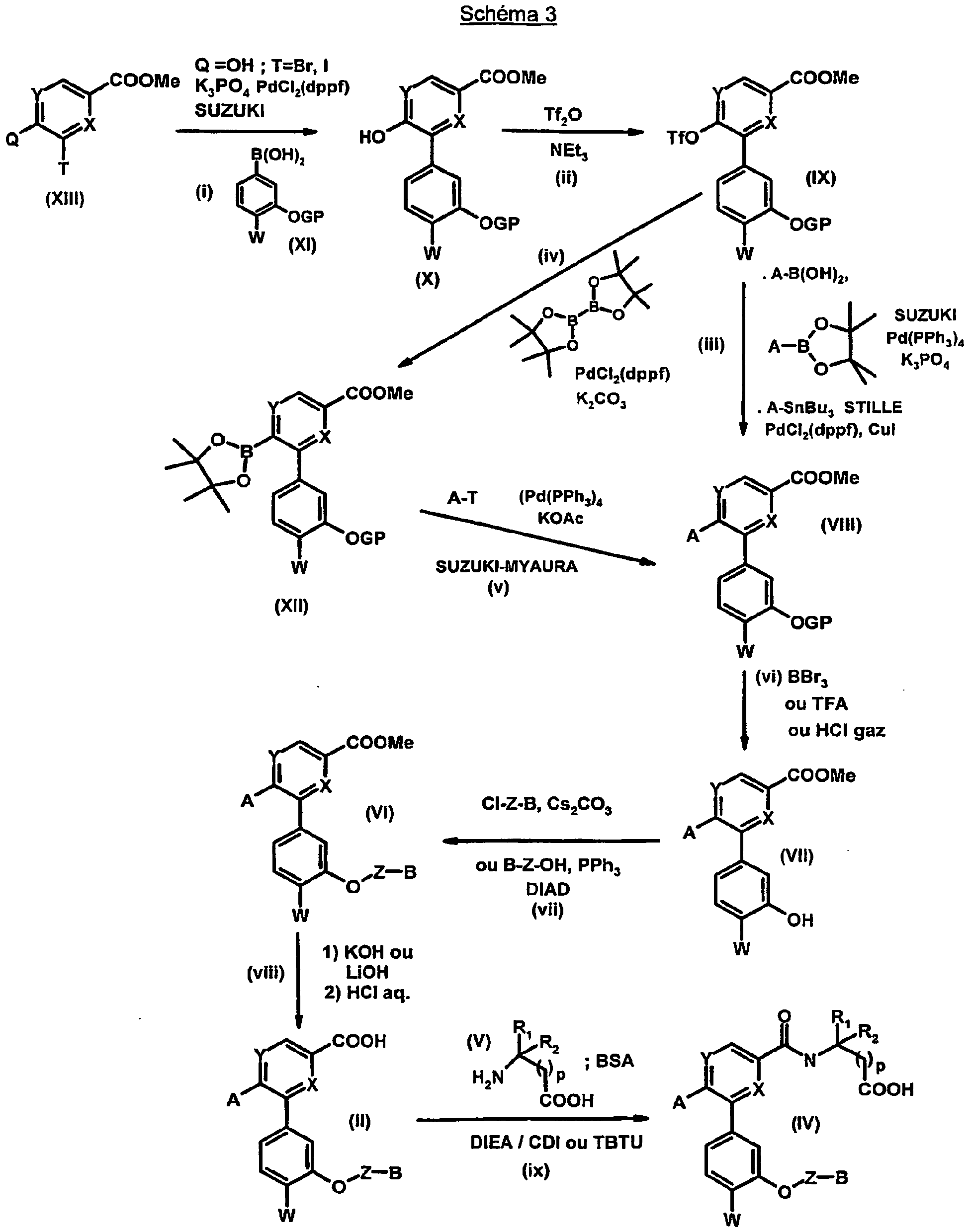 Dans les schémas 1, 2 et 3, les composés de départ et les réactifs, quand leurs modes de préparation ne sont pas décrits, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formules (II). Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans les tableaux ci-après, qui illustre les structures chimiques et les propriétés physiques de quelques composés selon l'invention.

Les abréviations et formules brutes suivantes sont utilisées :
- AcOEt: Acétate d'éthyle
- AcOH: Acide acétique
- BSA: N,O-Bis(triméthylsilyl)acétamide
- CDI: Carbonyldiimidazole
- Cul: Iodure de cuivre
- DCM: Dichlorométhane
- DIBAL-H: Hydrure de diisobutylaluminium
- DIAD: Azodicarboxylate de diisopropyle
- DIEA: N,N-diisopropyléthylamine
- DMAP: 4-Diméthylaminopyridine
- DME: Diméthoxyéthane
- DMF: Diméthylformamide
- DMSO: Dimethyl sulfoxide
- EDC HCl: Chlorhydrate de N-[3-(diméthylamino)propyl-N'-éthyl carbodiimide
- EtOH: Ethanol
- h: Heure(s)
- min: minute(s)
- HCI: Acide chlorhydrique
- K₂CO₃: Carbonate de potassium
- KOAc: Acétate de potassium
- KOH: Hydroxyde de sodium
- K₃PO₄: Phosphate de potassium ou tetraoxophosphate de tripotassium
- Na₂CO₃: Carbonate de sodium
- NH₄Cl: Chlorure d'ammonium
- NaHCO₃: Hydrogénocarbonate de sodium
- Na₂SO₄: Sulfate de sodium
- NMP: N-méthylpyrrolidinone
- PdCl₂ (dppf): Dichlorure de[1,1'-bis(cyclopentadiènyldiphénylphosphino)ferrocène] palladium (II)
- Pd(PPh₃)₄: Tetrakis (triphénylphosphine) palladium (0)
- Pd₂(dba)₃: Tris(dibenzylidèneacétone)dipalladium (0)
- TBTU: Tétrafluoroborate de N-[(1H-benzotriazol-1-yloxy)(diméthylamino)méthylidène]-N-méthylméthanaminium.
- TEA: Triéthylamine
- TFA: Acide trifluoroacétique
- THF: Tétrahydrofurane
- T.A.: Température ambiante.

Les spectres de masse sont obtenus dans les conditions de couplage LC/MS suivantes : Colonne :Kromasil 50x2,1 mm 6,5 µm
Eluants : A = CH₃CN / TFA (1000 / 0,5)
B = H₂O / CH₃CN / TFA (1000 / 30 / 0,5)

| Gradients | t (mm) | %A | %B | Débit (ml/mm) |
|---|---|---|---|---|
| | 0 | 0 | 100 | 0,5 |
| | 12 | 100 | 0 | 0,5 |
| | 15 | 100 | 0 | 0,5 |

On note le temps de rétention par Tr.

Les spectres de résonnance magnétique du proton (RMN¹H), tels que décrits ci-dessous, sont enregistrés à 400MHz dans du DMSO-d6, en utilisant le pic du DMSO-d5 comme référence. Les déplacements chimiques δ sont exprimés en partie par million (ppm). Les signaux observés sont exprimés ainsi : s = singulet ; d = doublet ; t = triplet ; m = massif ou singulet large ; H = proton.

### Exemple 1 : Chlorhydrate de 1-{[(3,5-dichloro-2'-{4-chloro-3-[3-(diméthylamino)propoxy]phényl}-2,3'-bipyridin-6'-yl)carbonyl]amino}cyclohexanecarboxylate de méthyle (composé N°1)

La synthèse du 6-bromo-5-hydroxy-2-pyridine carboxylate de méthyle est réalisée selon un procédé déjà décrit dans la littérature (J. Org. Chem., 1996, 4623-4633).

### 1.1 2-[(4-méthoxybenzyl)oxyl-1-chloro-4-iodobenzène

Une suspension de 300 g (1179 mmoles) de 2-chloro-5-iodophénol, de 184 g (1179 mmoles) de chlorure de 4-méthoxybenzyle et de 195,5 g (1415 mmoles) de K₂CO₃ anhydre dans 1,2 L de DMF anhydre est agitée 5 h à 70°C puis ramenée à T.A.. Le milieu réactionnel est ensuite réparti dans 3 L d'un mélange éther/eau 2:1. La phase organique est lavée avec 2 x 1 L d'eau, séchée sur Na₂SO₄ et concentrée sous pression réduite et le résidu obtenu est concrétisé dans du pentane. On obtient ainsi 406 g de 2-[(4-méthoxybenzyl)oxy]-1-chloro-4-iodobenzène sous forme de poudre beige.
Rendement = 92 %
F(°C) = 72.

### 1.2 Acide [3-[(4-méthoxybenzyl)oxy]-4-chlorophényl]boronique

A une solution de 145 g (387 mmoles) de 2-[(4-méthoxybenzyl)oxy]-1-chloro-4-iodobenzène dans 1,2 L de THF anhydre placée sous argon et agitée à -50°C, sont ajoutés goutte à goutte 220 mL (440 mmoles) d'une solution d'iPrMgCl 2N dans le THF en maintenant la température entre -40 et -50°C. On laisse revenir le mélange réactionnel à -10°C et l'agitation est maintenue 1 h. On ajoute ensuite 94 mL (406 mmoles) de borate de triisopropyle puis on laisse revenir lentement le mélange réactionnel à 0°C. Après 2 h d'agitation, le mélange est traité avec 500 mL d'une solution aqueuse d'HCl 5N puis extrait avec de l'éther (2 x 600 mL). La phase organique est lavée avec 2 x 1 L d'eau, séchée sur Na₂SO₄ puis concentrée sous pression réduite. Le résidu obtenu est concrétisé dans 300 mL de pentane, filtré sur fritté et lavé avec 200 mL de pentane. On obtient ainsi 96 g d'acide [3-[(4-méthoxybenzyl)oxy]-4-chlorophényl]boronique sous forme de solide blanc.
Rendement = 84 %
F(°C) = 148 (décomposition).

### 1.3 6-[3-[(4-méthoxybenzyl)oxy]-4-chlorophényl]-5-hydroxypyridine-2-carboxylate de méthyle

Une solution de 80 g (345 mmoles) de 6-bromo-5-hydroxy-2-pyridine carboxylate de méthyle et 146 g (499 mmoles) d'acide [3-[(4-méthoxybenzyl)oxy]-4-chlorophényl]boronique dans 1600 mL de dioxane anhydre est agitée 15 min sous barbotage d'argon puis on ajoute 143 g (1034 mmoles) de K₂CO₃ anhydre et 14 g (17,2 mmoles) de PdCl₂(dppf). Le milieu réactionnel est agité 10 h à 95°C sous argon, refroidi à T.A. puis réparti à T.A. dans 2 L d'AcOEt et 350 mL d'une solution aqueuse d'HCl 1 N. La phase organique est lavée avec 300 mL d'une solution aqueuse d'HCl 1 N et 600 mL d'eau. Après séchage sur Na₂SO₄ et concentration sous pression réduite, le précipité est repris par 500 mL d'un mélange pentane / DCM 7:3, filtré et lavé avec 200 mL de pentane. On obtient ainsi 105 g de 6-[3-[(4-méthoxybenzyl)oxy]-4-chlorophényl]-5-hydroxypyridine-2-carboxylate de méthyle sous forme de poudre brune utilisée telle quelle à l'étape suivante
Rendement = 76 %
F(°C) = 202

### 1.4 6-[3-[(4-méthoxybenzyl)oxy]-4-chlorophényl]-5-{[(trifluorométhyl)sulfonyl]oxy}pyridine-2-carboxylate de méthyle

A un mélange de 115 g (287 mmoles) de 6-[3-[(4-méthoxybenzyl)oxy]-4-chlorophényl]-5-hydroxypyridine-2-carboxylate de méthyle dans 1200 mL de DCM sont ajoutés 121.3 mL (863 mmoles) de TEA. Le mélange se solubilise progressivement et est refroidi à -20°C sous argon. On ajoute goutte à goutte 54,5 mL (331 mmoles) d'anhydride trifluorométhanesulfonique en maintenant la température à -20°C. Après 3 h à -10°C, le milieu réactionnel est repris avec 1 L de DCM et lavé avec 2 x 1 L d'eau, séché sur Na₂SO₄ puis concentré sous pression réduite. Le résidu obtenu est purifié par chromatographie sur une colonne de gel de silice en éluant avec un gradient pentane / AcOEt de 0 à 30 % d'AcOEt. Après concentration sous pression réduite, on obtient 138 g de 6-[3-[(4-méthoxybenzyl)oxy]-4-chlorophényl]-5-{[(trifluorométhyl)sulfonyl]oxy}pyridine-2-carboxylate de méthyle sous forme de cristaux blanc.
Rendement = 90 %
F(°C) = 89.

### 1.5 6-[3[(4-méthoxybenzyl)oxy]-4-chlorophényl]-5-(4,4,5,5-tétraméthyl-1,3,2-dioxaborolan-2-yl)pyridine-2-carboxylate de méthyle

Une solution de 30 g (56 mmoles) de 6-[3-[(4-méthoxybenzyl)oxy]-4-chlorophényl]-5-{[(trifluorométhyl)sulfonyl]oxy}pyridine-2-carboxylate de méthyle et de 28,6 g (113 mmoles) de 4,4,4',4',5,5,5',5'-octaméthyl-2,2'-bi-1,3,2-dioxaborolane dans 500 mL de toluène anhydre est agitée 15 min sous barbotage d'argon puis on ajoute 16,6 g (169 mmoles) de KOAc anhydre et 2,3 g (2,82 mmoles) de PdCl₂(dppf) et on chauffe le mélange réactionnel 26 h à 110°. Le mélange réactionnel est ensuite réparti dans 800 mL d'un mélange AcOEt/saumure 1:1. La phase organique est séchée sur Na₂SO₄, filtrée puis concentrée sous pression réduite. Le résidu obtenu est purifié par chromatographie sur une colonne de gel de silice en éluant avec un gradient DCM / AcOEt de 0 à 30 % d'AcOEt. Après concentration sous pression réduite, on obtient 22.4 g de 6-[3-[(4-méthoxybenzyl)oxy]-4-chlorophényl]-5-(4,4,5,5-tétraméthyl-1,3,2-dioxaborolan-2-yl)pyridine-2-carboxylate de méthyle sous forme d'une cire.
Rendement = 78 %

### 1.6 2'-[3-[(4-méthoxybenzyl)oxy]-4-chlorophényl]-3,5-dichloro-2,3'-bipyridine-6'-carboxylate de méthyle

A une suspension de 1 g (1,96 mmoles) de 6-[3-[(4-méthoxybenzyl)oxy]-4-chlorophényl]-5-(4,4,5,5-tétraméthyl-1,3,2-dioxaborolan-2-yl)pyridine-2-carboxylate de méthyle et 0,58 g (2,55 mmoles) de 2-bromo-3,5-dichloropyridine dans 10 mL d'un mélange DME/eau 2 :1, on fait barboter de l'argon pendant 10 min puis on ajoute successivement 0,81 g (5,88 mmoles) de K₂CO₃ et 0,159 g (0,14 mmole) de Pd(PPh₃)₄. Le mélange réactionnel est chauffé 4h à 80°C puis refroidi et dilué dans 50 mL d'AcOEt. La phase organique est lavée avec 2X20 mL d'eau, séchée sur Na₂SO₄ puis concentrée sous pression réduite. Le résidu est ensuite purifié sur une colonne de gel de silice en éluant avec un gradient cyclohexane/AcOEt de 0 à 40% d'AcOEt ce qui conduit à 0,4 g de 2'-[3-[(4-méthoxybenzyl)oxy]-4-chlorophényl]-3,5-dichloro-2,3'-bipyridine-6'-carboxylate de méthyle sous forme d'huile.
Rendement = 38 %

### 1.7 3,5-dichloro-2-(4-chloro-3-hydroxyphényl)-2,3'-bipyridine-6'-carboxylate de méthyle

A une solution de 0,4 g (0,75 mmoles) de 2'-[3-[(4-méthoxybenzyl)oxy]-4-chlorophényl]-3,5-dichloro-2-3'-pyridine-6'-carboxylate de méthyle dans 8 mL de DCM anhydre refroidie sous argon à 0°C sont ajoutés 0,6 mL de TFA. Après 2 h d'agitation à T.A., le milieu réactionnel est concentré sous pression réduite. Le résidu obtenu est repris par 10 mL d'AcOEt et 10 mL d'une solution aqueuse saturée de NaHCO₃. Après extraction, la phase aqueuse est re-extraite avec 10 mL d'AcOEt. Les phases organiques sont réunies, lavées avec 10 mL d'eau, séchées sur Na₂SO₄ et concentrées sous pression réduite. Le résidu est purifié par chromatographie sur une colonne de gel de silice en éluant avec un gradient heptane / AcOEt de 0 à 30 % en AcOEt. Après concentration sous pression réduite, on obtient 0,27 g de 3,5-dichloro-2'-(4-chloro-3-hydroxyphényl)-2,3'-bipyridine-6'-carboxylate de méthyle_sous forme d'huile.
Rendement = 89 %

### 1.8 3,5-dichloro-2'-{4-chloro-3-[3-(diméthylamino)propoxy]phényl}-2,3'-bipyridine-6'-carboxylate de méthyle

A une solution de 2g (4,88 mmoles) de 3,5-dichloro-2'-(4-chloro-3-hydroxyphényl)-2,3'-bipyridine-6'-carboxylate de méthyle dans 20 mL de DMF anhydre placée sous argon sont ajoutés 1,15 g (7,32 mmoles) de chlorhydrate de 3-chloro-N,N-diméthylpropane-1-amine et 4,77 g (14,64 mmoles) de carbonate de césium. Le milieu réactionnel est agité 18 h à 90°C, refroidi à T.A, puis concentré sous pression réduite. Le résidu est repris par 250 mL d'AcOEt et lavé avec 250 mL d'une solution aqueuse à 5 % de Na₂CO₃ puis 100 mL d'eau. Après séchage sur Na₂SO₄ et concentration sous pression réduite, le résidu est purifié par chromatographie sur une colonne de gel de silice en éluant avec un gradient DCM/méthanol de 0 à 15% de méthanol. Après concentration sous pression réduite, on obtient 1,82g de 3,5-dichloro-2'-{4-chloro-3-[3-(diméthylamino)propoxy]phényl}-2,3'-bipyridine-6'-carboxylate de méthyle sous forme de gomme.
Rendement = 80 %

### 1.9 Acide 3,5-dichloro-2'-{4-chloro-3-[3-(diméthylamino)propoxy]phényl}-2,3'-bipyridine-6'-carboxylique

A une solution de 1,82 g (3,9 mmoles) de 3,5-dichloro-2'-{4-chloro-3-[3-(diméthylamino)propoxy]phényl}-2,3'-bipyridine-6'-carboxylate de méthyle dans 50 mL d'EtOH et 10 mL d'eau sont ajoutés 0,34 g (6,06 mmoles) de KOH. Le milieu réactionnel est agité 18 h à T.A. puis concentré sous pression réduite. Le résidu obtenu est repris par 6,1 mL d'une solution aqueuse d'HCl 1N (6,1 mmoles) et 100 mL d'un mélange DCM/MeOH 9 :1. La phase organique est séchée sur Na₂SO₄ puis concentrée sous pression réduite, ce qui permet d'obtenir 1,8 g d'acide 3,5-dichloro-2'-{4-chloro-3-[3-(diméthylamino)propoxy]phényl}-2,3'-bipyridine-6'-carboxylique sous forme de gomme. Rendement = 95 %

### 1.10 Chlorhydrate d'acide 1-{[(3.5-dichloro-2'-{4-chloro-3-[3-(diméthylamino)propoxy]phényl}-2,3'-bipyridin-6'-yl)carbonyl]amino}cyclohexanecarboxylique

A une solution de 1,49 g (3,1 mmoles) d'acide 3,5-dichloro-2'-{4-chloro-3-[3-(diméthylamino)propoxy]phényl}-2,3'-bipyridine-6'-carboxylique dans 15mL de NMP anhydre sont ajoutés sous argon 1,62 mL (9,3 mmoles) de DIEA et 2,83 g (8,82 mmoles) de TBTU. Parallèlement, on porte à 90°C sous agitation et sous argon un mélange de 421 mg (2,94 mmoles) d'acide 1-aminocyclohexane carboxylique et de 1,51 mL (6,19 mmoles) de BSA dans 15 mL d'acétonitrile anhydre. Après 2 h, le milieu se solubilise complètement et la solution est ramenée à T.A. puis ajoutée à la solution d'acide 3,5-dichloro-2'-{4-chloro-3-[3-(diméthylamino)propoxy]phényl}-2,3'-bipyridine-6'-carboxylique activé avec TBTU. Après 2 h d'agitation à T.A., on ajoute 10 mL d'une solution aqueuse 0,5 N d'HCl et l'agitation est maintenue 18 h. Le milieu réactionnel est alors réparti dans un mélange de 100 mL de DCM/MeOH 9:1 et 10 mL d'eau. Après extraction, la phase aqueuse est à nouveau extraite avec 10 mL d'un mélange DCM/MeOH 9:1. Les phases organiques sont réunies, séchées sur Na₂SO₄ et concentrée sous pression réduite. Le résidu est ensuite purifié par HPLC sur phase inverse (RP18) en éluant avec un gradient HCl aqueux 10⁻²N / acétonitrile de 5 % à 100 % d'acétonitrile. Après concentration sous pression réduite et une lyophilisation, on obtient 1 g de chlorhydrate d'acide 1-{[(3,5-dichloro-2'-{4-chloro-3-[3-(diméthylamino)propoxy]phényl}-2,3'-bipyridin-6'-yl)carbonyl]amino}cyclohexanecarboxylique sous forme de poudre blanche.
Rendement = 50 %
F(°C) = 165

### 1.11. Chlorhydrate de 1-{[(3,5-dichloro-2'-{4-chloro-3-[3-(diméthylamino)propoxy]phényl}-2,3'-bipyridin-6'-yl)carbonyl]amino}cyclohexanecarboxylate de méthyle

A une solution de 300 mg (0,47 mmole) de chlorhydrate d'acide 1-{[(3,5-dichloro-2'-{4-chloro-3-[3-(diméthylamino)propoxy]phényl}-2,3'-bipyridin-6'-yl)carbonyl]amino}cyclohexanecarboxylique dans 10 mL de méthanol et refroidie à 0°C sont ajoutés 0,07 mL (0,93 mmole) de chlorure de thionyle. Le mélange réactionnel est ramené à T.A. et agité pendant 20 h puis concentré sous pression réduite. Le résidu obtenu est repris par 20 mL d'éther et concrétisé. Après séchage sous pression réduite, on obtient 268 mg de chlorhydrate de 1-{[(3,5-dichloro-2'-{4-chloro-3-[3-(diméthylamino)propoxy]phényl}-2,3'-bipyridin-6'-yl)carbonyl]amino}cyclohexanecarboxylate de méthyle sous forme de poudre blanche.
Rendement =87 %
F(°C) = 168
M = C₃₀H₃₃Cl₃N₄O₄ = 618 ; M+H = 619 ; Tr = 1,14 min.
RMN¹H (ppm, d6-DMSO, 400 MHz) : 9,90 (s, 1H) ; 8,80 (s, 1H) ; 8,65 (s, 1 H) ; 8,35 (s, 1H) ; 8,20 (d, 1H) ; 8,15 (d, 1H) ; 7,40 (d, 2H) ; 6,80 (d, 1H) ; 4,05 (m, 2H) ; 3,65 (s, 3H) ; 3,20 (m, 2H) ; 2,80 (s, 6H) ; 2,20 (m, 4H) ; 1,85 (t, 2H) ; 1,7 - 1,3 (m, 6H).

### Exemple 2 : Chlorhydrate de (3S)-3-({[6-{4-chloro-3-[3-(diméthylamino)propoxy]phényl}-5-(2-chlorophényl)pyridin-2-yl]carbonyl}amino)-4,4-diméthylpentanoate de méthyle (composé N°31)

### 2.1 Acide 6-{4-chloro-3-[3-(diméthylamino)propoxy]phényl}-5-(2-chlorophényl)pyridine-2-carboxylate de méthyle

Selon la séquence débenzylation/O-alkylation/saponification décrite dans les exemples 1.7, 1.8 et 1.9 respectivement, on obtient, à partir de 665 mg (1,35 mmoles) de 6-[3-[(4-méthoxybenzyl)oxy]-4-chlorophényl]-5-(2-chlorophényl)pyridine-2-carboxylate de méthyle, 181 mg de chlorhydrate d'acide 6-{4-chloro-3-[3-(diméthylamino)propoxy]phényl}-5-(2-chlorophényl)pyridine-2-carboxylique sous forme de gomme.
Rendement = 28 %

### 2.2 Chlorhydrate d'acide (3S)-3-({[6-{4-chloro-3-[3-(diméthylamino)propoxy]phényl}-5-(2-chlorohényl)pyridin-2-yl]carbonyl}amino)-4.4-diméthylpentanoique

A un mélange de 674 mg (1,4 mmoles) de chlorhydrate d'acide 6-{4-chloro-3-[3-(diméthylamino)propoxy]phényl}-5-(2-chlorophényl)pyridine-2-carboxylique dans 7 mL de DMF anhydre, on ajoute successivement sous argon et à T.A., 240 mg (2,1 mmoles) de N-hydroxysuccinimide et 401 mg (2,1 mmoles) d'EDC.HCl. Après 18 h d'agitation on ajoute successivement au milieu réactionnel 1,31 mL (7,7 mmoles) de DIEA et 304 mg (1,51 mmoles) de (3S)-3-amino-4,4-diméthylpentanoate de tert-butyle (J.Org.Chem., 1999, 64, 6411-6417). L'agitation est poursuivie 18 h à T.A. puis le mélange réactionnel est concentré sous pression réduite. Le résidu obtenu est ensuite traité 18 h à T.A. avec 0,25 mL (3 mmoles) d'une solution aqueuse d'HCl 12N dans 30 mL d'acide formique puis concentré sous pression réduite. Après purification par HPLC sur phase inverse (RP18) en éluant avec un gradient HCl aqueux 10⁻²N / acétonitrile de 5 % à 100 % d'acétonitrile, on obtient, après lyophilisation, 500 mg de chlorhydrate d'acide (3S)-3-({[6-{4-chloro-3-[3-(diméthylamino)propoxy]phényl}-5-(2-chlorophényl)pyridin-2-yl]carbonyl}amino)-4,4-diméthylpentanoique sous forme de poudre blanche.
Rendement = 59 %
F (°C) = 170

### 2.3. Chlorhydrate de (3S)-3-({[6-{4-chloro-3-[3-(diméthylamino)propoxy]phényl}-5-(2-chlorophényl)pyridin-2-yl]carbonyl}amino)-4,4-diméthylpentanoate de méthyle

Selon le mode opératoire décrit dans l'exemple 1.11, l'estérification de 217 mg (0,356 mmoles) de chlorhydrate d'acide (3*S*)-3-({[6-{4-chloro-3-[3-(diméthylamino)propoxy]phényl}-5-(2-chlorophényl)pyridin-2-yl]carbonyl}amino)-4,4-diméthylpentanoique avec 0,05 mL (0,71 mmoles) de chlorure de thionyle dans 4 mL de méthanol conduit, après concrétisation dans 20 mL d'éther et une lyophilisation, à 196 mg de chlorhydrate de (3*S*)-3-({[6-{4-chloro-3-[3-(diméthylamino)propoxy]phényl}-5-(2-chlorophényl)pyridin-2-yl]carbonyl}amino)-4,4-diméthylpentanoate de méthyle sous forme de poudre blanche.
Rendement = 94 %
F(°C) = 126
[α]_{D}²⁰ = -17° (c = 0,1, MeOH)
M = C₃₁H₃₇Cl₂N₃O₄ = 585 ; M+H = 586 ; Tr = 1,14 min.
RMN¹H (ppm, d6-DMSO, 400 MHz) : 10,15 (s, 1H) ; 8,45 (dd, 1H) ; 8,10 (dd, 1H) ; 8,05 (d, 1H) ; 7,55 (m, 1H) ; 7,45 (m, 3H) ; 7,40 (d, 1H) ; 7,20 (d, 1H) ; 6,95 (dd, 1H) ; 4,35 (td, 1H) ; 3,95 (m, 2H) ; 3,55 (d, 3H) ; 3,20 (m, 2H) ; 2,80 (d, 6H) ; 2,70 (m, 2H) ; 2,15 (m, 2H) ; 0,95 (d, 9H).

### Exemple 3 : Chlorhydrate de 1-{[(3-chloro-2'-{4-chloro-3-[3-(diméthylamino)propoxy]phényl}-5-méthyl-2,3'-bipyridin-6'-yl)carbonyl]amino}cyclohexanecarboxylate de méthyle (composé N°5)

### 3.1 3-chloro-2'-{4-chloro-3-[(4-méthoxybenzyl)oxy]phényl}-5-méthyl-2,3'-bipyridine-6'-carboxylate de méthyle

Le couplage de Suzuki-Myaura effectué entre 1 g (1,96 mmoles) de 6-[3-[(4-méthoxybenzyl)oxy]-4-chlorophényl]-5-(4,4,5,5-tétraméthyl-1,3,2-dioxaborolan-2-yl)pyridine-2-carboxylate de méthyle et 526 mg (2,55 mmoles) de 2-bromo-3-chloro-5-méthylpyridine selon l'exemple 1.6 conduit à 637 mg de 3-chloro-2'{4-chloro-3-[(4-méthoxybenzyl)oxy]phényl}-5-méthyl-2,3'-bipyridine-6'-carboxylate de méthyle sous forme d'huile.
Rendement = 64 %

### 3.2 Acide 3-chloro-2'-{4-chloro-3-[3-(diméthylamino)propoxy]phényl}-5-méthyl-2,3'-bipyridine-6'-carboxylique

Selon la séquence débenzylation/O-alkylation/saponification décrite dans les exemples 1.7., 1.8. et 1.9. respectivement, on obtient à partir de 338 mg (0,66 mmole) de 3-chloro-2'-{4-chloro-3-[(4-méthoxybenzyl)oxy]phényl}-5-méthyl-2,3'-bipyridine-6'-carboxylate de méthyle, 134 mg d'acide 3-chloro-2'-{4-chloro-3-[3-(diméthylamino)propoxy]phényl}-5-méthyl-2,3'-bipyridine-6'-carboxylique sous forme de gomme brune.
Rendement = 45 %

### 3.3. Chlorhydrate de 1-aminocyclohexane carboxylate de méthyle

A une suspension de 2 g (14 mmoles) d'acide 1-aminocyclohexane carboxylique dans 20 mL de méthanol, on fait barboter du chlorure d'hydrogène pendant 2 minutes. A la solution obtenue, on ajoute 1,2 mL (16,8 mmoles) de chlorure de thionyle et le milieu réactionnel est porté 6 h à reflux. Après refroidissement, le mélange et concentré sous pression réduite et le résidu obtenu est repris par 5 mL de méthanol. On ajoute 50 mL d'éther afin de précipiter 2,3 g de chlorhydrate de 1-aminocyclohexane carboxylate de méthyle sous forme de poudre blanche.
Rendement = 85 %
F(°C) = 240

### 3.4. Chlorhydrate de 1-{[(3-chloro-2'-{4-chloro-3-[3-(diméthylamino)propoxy]phényl}-5-méthyl-2,3'-bipyridin-6'-yl)carbonyl]amino}cyclohexanecarboxylate de méthyle

A une suspension de 134 mg (0,29 mmoles) d'acide 3-chloro-2'-{4-chloro-3-[3-(diméthylamino)propoxy]phényl}-5-méthyl-2,3'-bipyridine-6'-carboxylique dans 5 mL d'acétonitrile anhydre sont ajoutés successivement 0,18 mL (1,02 mmoles) de DIEA et 56 mg (0,29 mmoles) de chlorhydrate de 1-amino-cyclohexane carboxylate de méthyle. Le mélange est refroidi à 0°C et on ajoute 112 mg (0,35 mmole) de TBTU. Après 18 h d'agitation entre 0 et 10°C, le milieu réactionnel est concentré sous pression réduite puis repris dans 20 mL d'un mélange AcOEt/éther 1 :1. Après lavage avec 10 mL d'eau et 10 mL d'une solution aqueuse saturée de NaHCO₃, la phase organique est séchée sur Na₂SO₄ et concentrée sous pression réduite. Au résidu repris par 5 mL de DCM, on ajoute 0,2 mL (0,1 mmoles) d'une solution d'HCl 2N dans l'éther puis 20 mL d'éther anhydre ce qui permet de précipiter 139 mg de chlorhydrate de 1-{[(3-chloro-2'-{4-chloro-3-[3-(diméthylamino)propoxy]phényl}-5-méthyl-2,3'-bipyridin-6'-yl)carbonyl]amino}cyclohexanecarboxylate de méthyle sous forme de poudre blanche.
Rendement = 80 %
F (°C) = 152
M = C₃₁H₃₆Cl₂N₄O₄ = 598 ; M+H = 599 ; Tr = 1,11 min.
RMN¹H (ppm, d6-DMSO, 400 MHz) : 10,25 (s, 1H) ; 8,60 (s, 1H) ; 8,52 (s, 1H) ; 8,10 (t, 2H) ; 7,85 (s, 1H) ; 7,40 (d, 1H) ; 7,35 (d, 1H) ; 6,90 (dd, 1H) ; 4,00 (m, 2H) ; 3,65 (s, 3H) ; 3,20 (m, 2H) ; 2,80 (d, 6H) ; 2,40 (s, 3H) ; 2,20 (m, 4H) ; 1,90 (t, 2H) ; 1,70- 1,30 (m, 6H).

### Exemple 4 : Chlorhydrate de 1-({[6-{4-chloro-3-[3-(diméthylamino)propoxy]phényl}-5-(2-méthylphényl)pyridin-2-yl]carbonyl}amino)cyclohexanecarboxylate de méthyle (composé N°4)

### 4.1. 6-[3-[(4-méthoxvbenzyl)]oxy-4-chlorophényl]-5-(2-méthylphényl)pyridine-2-carboxylate de méthyle

Une solution de 2,6 g (4,88 mmoles) de 6-[3-[(4-méthoxybenzyl)oxy]-4-chlorophényl]-5-{[(trifluorométhyl)sulfonyl]oxy}pyridine-2-carboxylate de méthyle *(exemple 1.4)* et de 880 mg (6,48 mmoles) d'acide 2-méthylphénylboronique dans 20 mL de DMF anhydre est agitée 15 min sous barbotage d'argon puis on ajoute 1,27 g (6 mmoles) de K₃PO₄ anhydre et 0,58 g (0,5 mmoles) de Pd(PPh₃)₄ et le mélange réactionnel est agité 18 h à 90°C sous argon. Le milieu réactionnel est ensuite réparti à T.A. dans 120 mL d'un mélange éther/AcOEt/eau 1:1:2. Après extraction, la phase aqueuse est re-extraite avec 10 mL d'AcOEt, les phases organiques sont réunies et lavées avec 4 x 30 mL d'eau, séchées sur Na₂SO₄ puis concentrées sous pression réduite. Le résidu obtenu est purifié par chromatographie sur une colonne de gel de silice en éluant avec un gradient heptane / AcOEt de 0 à 20 % d'AcOEt. Après concentration sous pression réduite, on obtient 1,8 g de 6-[3-[(4-méthoxybenzyl)]oxy-4-chlorophényl]-5-(2-méthylphényl)pyridine-2-carboxylate de méthyle sous forme d'huile.
Rendement = 81 %

### 4.2. Acide 6-{4-chloro-3-[3-(diméthylamino)propoxy]phényl}-5-(2-méthylphényl)pyridine-2-carboxylique.

Selon la séquence débenzylation/O-alkylation/saponification décrite dans les exemples 1.7, 1.8 et 1.9 respectivement, on obtient, à partir de 5,3 g (11 mmoles) de 6-[3-[(4-méthoxybenzyl)]oxy-4-chlorophényl]-5-(2-méthylphényl)pyridine-2-carboxylate de méthyle et de 1,4 g (8,9 mmoles) de chlorhydrate de 3-chloro-N,N-diméthylpropane-1-amine, 3,9 g d'acide 6-{4-chloro-3-[3-(diméthylamino)propoxy]phényl}-5-(2-méthylphényl)pyridine-2-carboxylique sous forme de gomme.
Rendement = 81 %
F (°C) = 146-150

### 4.3. Chlorhydrate de 1-({[6-{4-chloro-3-[3-(diméthylamino)propoxy]phényl}-5-(2-méthylphényl)pyridin-2-yl]carbonyl}amino)cyclohexanecarboxylate de méthyle

Selon le mode opératoire décrit dans l'exemple 3.5, le couplage peptidique effectué entre 300 mg (0,71 mmole) d'acide 6-{4-chloro-3-[3-(diméthylamino)propoxy]phényl}-5-(2-méthylphényl)pyridine-2-carboxylique et 137 mg (0,71 mmole) de chlorhydrate de 1-aminocyclohexane carboxylate de méthyle conduit, après concrétisation, à 190 mg de chlorhydrate de 1-({[6-{4-chloro-3-[3-(diméthylamino)propoxy]phényl}-5-(2-méthylphényl)pyridin-2-yl]carbonyl}amino)cyclohexanecarboxylate de méthyle sous forme de poudre blanche.
Rendement = 48 %
F (°C) = 115
M = C₃₂H₃₈ClN₃O₄ = 563 ; M+H = 564 ; Tr = 1,18 min.
RMN¹H (ppm, d6-DMSO, 400 MHz) : 10,2 (s, 1H) ; 8,55 (s, 1H) ; 8,10 (d, 1H) ; 7,95 (d, 1H) ; 7,40-7,20 (m, 6H) ; 7,05 (dd, 1H) ; 3,85 (m, 2H) ; 3,65 (s, 3H) ; 3,20 (m, 2H) ; 2,80 (d, 6H) ; 2,20 (d, 2H) ; 2,10 (m, 2H) ; 1,90 (s, 3H) ; 1,85 (t, 2H) ; 1,70 - 1,30 (m, 6H).

### Exemple 5 : Chlorhydrate de cis-1-({[6-{4-chloro-3-[3-(diméthylamino)propoxy]phényl}-5-(2-méthylphényl)pyridin-2-yl]carbonyl}amino)-4-hydroxycyclohexanecarboxylate de méthyle (composé N°42)

### 5.1. Chlorhydrate de cis-1-amino-4-hydroxycyclohexane carboxylate de méthyle

Selon le mode opératoire décrit dans l'exemple 3.4, l'estérification de 1,41 g (7,21 mmoles) d'acide cis-1-amino-4-hydroxyclyclohexane carboxylique *(*J.Chem. Soc., Perkin Trans. 1 (1999) pp 3375-3379*)*, avec 0,62 mL (8,65 mmoles) de chlorure de thionyle dans 30 mL de méthanol conduit à 1,5 g de chlorhydrate de cis-1-amino-4-hydroxycyclohexane carboxylate de méthyle sous forme de poudre blanche.
Rendement = 100 %
F(°C) = 160.

### 5.2. Chlorhydrate de cis 1-({[6-{4-chloro-3-[3-(diméthylamino)propoxy]phényl}-5-(2-méthylphényl)pyridin-2-yl]carbonyl}amino)-4-hydroxycyclohexanecarboxylate de méthyle

A une suspension de 467 mg (1,1 mmoles) d'acide 6-{4-chloro-3-[3-(diméthylamino)propoxy]phényl}-5-(2-méthylphényl)pyridine-2-carboxylique *(exemple 4.2)* dans 11 mL de DMF anhydre sont ajoutés successivement sous argon et à T.A. 139 mg (1,21 mmoles) de N-hydroxy succinimide et 232 mg (1,21 mmoles) d'EDC.HCl. Le mélange réactionnel est agité 24 h à T.A. puis on ajoute à la solution jaune et limpide 253 mg (1,21 mmoles) de chlorhydrate de *cis*-1-amino-4-hydroxycyclohexane carboxylate de méthyle et 1 mL (5,94 mmoles) de DIEA. Après à nouveau 16 h d'agitation à T.A., le milieu réactionnel est dilué dans 30 mL d'AcOEt et la phase organique est lavée avec 3 x 20 mL d'eau. Après séchage sur Na₂SO₄ et concentration sous pression réduite, le résidu obtenu est repris par un mélange de 5 mL de méthanol et 1,5 mL d'une solution aqueuse d'HCl 1N (1,5 mmoles) puis purifié par HPLC sur phase inverse (RP18) en éluant avec un gradient HCl aqueux 10⁻² N/acétonitrile de 5 % à 100 % d'acétonitrile en 90 minutes. Après concentration sous pression réduite et lyophilisation, on obtient 252 mg de chlorhydrate de *cis* 1-({[6-{4-chloro-3-[3-(diméthylamino)propoxy]phényl}-5-(2-méthylphényl)pyridin-2-yl]carbonyl}amino)-4-hydroxycyclohexanecarboxylate de méthyle sous forme de poudre blanche.
Rendement = 37 %
F (°C) = 244°C
M = C₃₂H₃₈ClN₃O₅ = 579 ; M+H = 580 ; Tr = 7,2 min (gradient de 20 min.).
RMN¹H (ppm, d6-DMSO, 400 MHz) : 9,80 (s, 1H) ; 8,60 (s, 1H) ; 8, 05 (d, 1H) ; 7,95 (d, 1H) ; 7,35 (d, 1H) ; 7,30-7,10 (m, 5H) ; 6,95 (dd, 1H) ; 4,75 (m, 1H) ; 3,95 (m, 2H) ; 3,65 (s, 3H) ; 3,55 (m, 1H) ; 3,20 (m, 2H) ; 2,85 (s, 6H) ; 2,30 (m, 2H) ; 2,15 (m, 2H) ; 1,90 (s, 3H) ; 1,80 (m, 4H) ; 1,40 (m, 2H).

### Exemple 6 : Chlorhydrate de 6-{4-chloro-3-[3-(diméthylamino)propoxy]phényl}-N-[(1S)-2,2-diméthyl-1-{2-[(méthylsulfonyl)amino]-2-oxoéthyl}propyl]-5-(2-méthylphényl)pyridine-2-carboxamide (composé N°34)

### 6.1 Chlorhydrate d'acide (3S)-3-({[6-{4-chloro-3-[3-(diméthylamino)propoxy]phényl}-5-(2,4-diméthylphényl)pyridin-2-yl]carbonyl}amino)-4.4-diméthylpentanoique.

Selon le procédé décrit dans l'exemple 2.2, le couplage peptidique effectué entre 425 mg (1 mmoles) d'acide 6-{4-chloro-3-[3-(diméthylamino)propoxy]phényl}-5-(2-méthylphényl)pyridine-2-carboxylique *(exemple 4.2)* et 206 mg (2,05 mmoles) de (3S)-3-amino-4,4-diméthylpentanoate de tert-butyle (J.Org.Chem. , 1999, 64, 6411-6417) conduit à 390 mg de chlorhydrate d'acide (3*S*)-3-({[6{4-chloro-3-[3-(diméthylamino)propoxy]phényl}-5-(2-méthylphényl)pyridin-2-yl]carbonyl}amino)-4,4-diméthylpentanoique sous forme de poudre blanche.
Rendement = 66%
F(°C) = 130
[α]_{D}²² = -5° ; (c = 0,1 ; MeOH)

### 6.2 Chlorhydrate de 6-{4-chloro-3-[3-(diméthylamino)propoxy]phényl}-N-[(1S)-2,2-diméthyl-1-{2-[(méthylsulfonyl)amino]-2-oxoéthyl}propyl]-5-(2-méthylphényl)pyridine-2-carboxamide

A une solution de 250 mg (0,42 mmole) de chlorhydrate d'acide (3*S*)-3-({[6-{4-chloro-3-[3-(diméthylamino)propoxy]phényl}-5-(2-méthylphényl)pyridin-2-yl]carbonyl}amino)-4,4-diméthylpentanoique et 49 mg (0,51 mmole) de méthanesulfonamide dans 2 mL de DCM sont successivement ajoutés 52 mg (0,42 mmole) de DMAP et 89 mg (0,47 mmole) d'EDC.HCl. Le milieu réactionnel est agité 16 h à T.A.. On ajoute à nouveau 0,42 mmole d'EDC.HCl et 0,42 mmole de méthanesulfonamide et le mélange réactionnel est agité pendant encore 3 jours puis concentré sous pression réduite. Le résidu est repris par 100 mL d'AcOEt et la phase organique est lavée avec 30 mL d'eau puis 30 mL d'une solution aqueuse 4M de NaHCO₃. Après séchage sur Na₂SO₄ et concentration sous pression réduite, le résidu est purifié par chromatographie sur une colonne de gel de silice en éluant avec un gradient DCM/méthanol de 0 à 10% en méthanol. Après concentration sous pression réduite, le résidu est repris par 2 mL de méthanol et 0,5 mL d'une solution aqueuse d'HCl1N puis purifié par HPLC sur phase inverse (RP18) en éluant avec un gradient HCl 10⁻² N: acétonitrile de 5 % à 100 % d'acétonitrile en 90 minutes. Après concentration sous pression réduite et une lyophilisation, on obtient 130 mg de chlorhydrate de 6-{4-chloro-3-[3-(diméthylamino)propoxy]phényl}-*N*-[(1*S*)-2,2-diméthyl-1-{2-[(méthylsulfonyl)amino]-2-oxoéthyl}propyl]-5-(2-méthylphényl)pyridine-2-carboxamide sous forme de poudre blanche.
Rendement = 46 %
F (°C) = 138 °C
[α]_{D}²² = -45° ; (c = 0,5 ; MeOH)
M = C₃₂H₄₁ClN₄O₅S = 628 ; M+H = 629 ; Tr =1,06 min.
RMN¹H (ppm, d6-DMSO, 400 MHz) : 11,80 (d, 1H) ; 10,30 (s, 1H) ; 8,40 (t, 1H) ; 8, 05 (d, 1H) ; 7,95 (d, 1H) ; 7,30 (m, 5H) ; 7,15 (d, 1H) ; 7,05 (dd, 1H) ; 4,35 (t, 1H) ; 3,80 (m, 2H) ; 3,15 (m, 2H) ; 3,05 (s, 3H) ; 2,80 (s, 6H) ; 2,65 (m, 2H) ; 2,10 (m, 2H) ; 1,90 (s, 3H) ; 1,00 (d, 9H).

### Exemple 7 : Chlorhydrate de 6-{4-chloro-3-[3-(diméthylamino)propoxy]phényl}-N-[(1S)-1-(2-hydroxyéthyl)-2,2-diméthylpropyl]-5-(2-méthylphényl)pyridine-2-carboxamide (composé N°37)

A une solution de 250 mg (0,42 mmole) de chlorhydrate d'acide (3*S*)-3-({[6-{4-chloro-3-[3-(diméthylamino)propoxy]phényl}-5-(2-méthylphényl)pyridin-2-yl]carbonyl}amino)-4,4-diméthylpentanoique *(exemple 6.1)* dans 2 mL de THF sont ajoutés goutte à goutte sous argon 1,06 mL (2,12 mmoles) d'une solution de complexe borane-diméthylsulfure 2N dans le THF. Le milieu réactionnel est agité 16 h à T.A. puis dilué dans 10 mL de méthanol anhydre. La solution obtenue est concentrée sous pression réduite et le résidu est repris par 20 mL de méthanol puis à nouveau concentré sous pression réduite. Le résidu obtenu est alors dissout dans 20 mL d'un mélange THF/solution aqueuse d'HCl 1N 1 :1 et la solution est portée 4 h à reflux. Après refroidissement et concentration sous pression réduite, le résidu est purifié par HPLC sur phase inverse (RP18) en éluant avec un gradient HCl 10⁻²N/acétonitrile de 0 à 100 % d'acétonitrile en 120 minutes. Après concentration sous pression réduite et lyophilisation, on obtient 50 mg de chlorhydrate de 6-{4-chloro-3-[3-(diméthylamino)propoxy]phényl}-*N*-[(1*S*)-1-(2-hydroxyéthyl)-2,2-diméthylpropyl]-5-(2-méthylphényl)pyridine-2-carboxamide sous forme de poudre blanche.
Rendement = 20 %
F(°C) = 93
[α]_{D}²⁰ = -20° (c = 0,4, MeOH)
M = C₃₁H₄₀ClN₃O₃ = 537 ; M+H = 538 ; Tr = 1,06 min.
RMN¹H (ppm, d6-DMSO, 400 MHz) : 10,10 (s, 1H) ; 8,15 (d, 1H) ; 8, 10 (dd, 1H) ; 7,95 (d, 1H) ; 7,30 (m, 5H) ; 7,10 (m, 1H) ; 7,05 (dd, 1H) ; 3,95 (t, 1H) ; 3,80 (m, 2H) ; 3,45 (m, 2H) ; 3,15 (m, 2H) ; 2,80 (d, 6H) ; 2,10 (m, 2H) ; 1,90 (d, 3H) ; 1,85 (m, 1H) ; 1,65 (m, 1H) ; 0,95 (d, 9H).

Les tableaux qui suivent illustrent les structures chimiques et les propriétés physiques de quelques exemples de composés selon l'invention.

Les composés décrits dans les tableaux ont tous été obtenus sous forme de chlorhydrate.

Le tableau 1 illustre des composés de formule (Ic) correspondant à des composés de formule (I) pour lesquels W représente un atome de chlore, Z représente -(CH₂)₃- et B représente -N(CH₃)₂.

Le tableau 2 illustre des composés de formule (Id) correspondant à des composés de formule (I) pour lesquels W représente un atome de chlore, Z représente -(CH₂)₃- et B représente -N(CH₃)₂, p est égal à 0 et R3 représente C(O)OMe.

Dans ces tableaux :
- Me, Et et i-Pr représentent respectivement des groupes méthyle, éthyle et isopropyle,
- C(O)OAlkyle représente une fonction ester par exemple C(O)OMe représente une fonction ester méthylique, C(O)OiPr représente une fonction ester isopropylique, etc,
- C(O)NH₂ représente une fonction amide, etc
- la colonne PF indique le point de fusion, en °C, du composé.

### TABLEAU DES COMPOSES

**TABLEAU 1**

| | | | | | | | | |
|---|---|---|---|---|---|---|---|---|
| 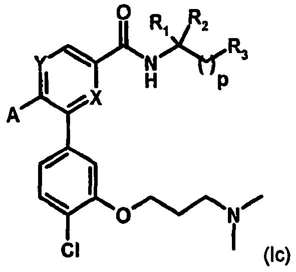 | | | | | | | | |

| N° | A | p | R₂ | R₁ | X | Y | R₃ | Point de fusion (°C) |
|---|---|---|---|---|---|---|---|---|
| 1 Ex1 | 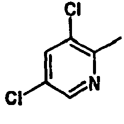 | 0 | 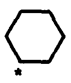 | | N | CH | C(O)OMe | 168 |
| 2 | 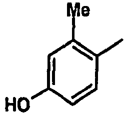 | 0 | 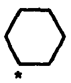 | | N | CH | C(O)OMe | 270 |
| 3 | 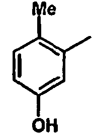 | 0 | 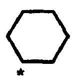 | | N | CH | C(O)OMe | 166 |
| 4 Ex 4 | 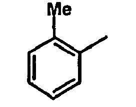 | 0 | 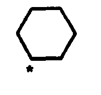 | | N | CH | C(O)OMe | 115 |
| 5 Ex 3 | 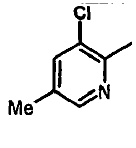 | 0 | 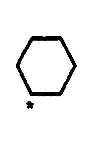 | | N | CH | C(O)OMe | 152 |
| 6 | 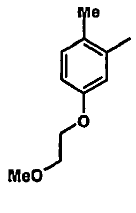 | 0 | 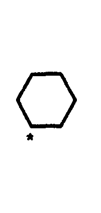 | | N | CH | C(O)OMe | 200 |
| 7 | 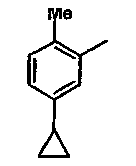 | 0 | 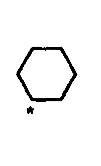 | | N | CH | C(O)OMe | 114 |
| 8 | 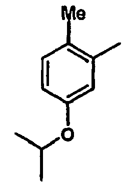 | 0 | 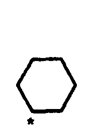 | | N | CH | C(O)OMe | 194 |
| 9 | 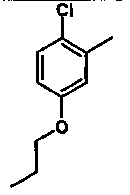 | 0 | 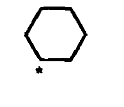 | | N | CH | C(O)OMe | 117 |
| 10 | 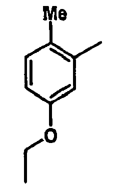 | 0 | 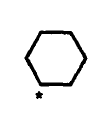 | | N | CH | C(O)OMe | 116 |
| 11 | 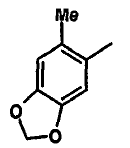 | 0 | 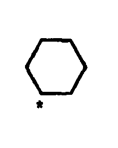 | | N | CH | C(O)OMe | 138 |
| 12 | 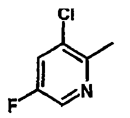 | 0 | 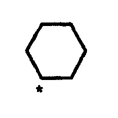 | | N | CH | C(O)OMe | 160 |
| 13 | 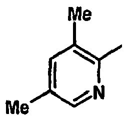 | 0 | 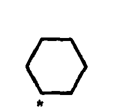 | | N | CH | C(O)OMe | 176 |
| 14 | 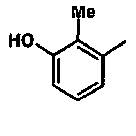 | 0 | 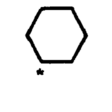 | | N | CH | C(O)OMe | >250 |
| 15 | 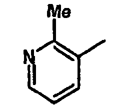 | 0 | 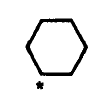 | | N | CH | C(O)OMe | 172 |
| 16 | 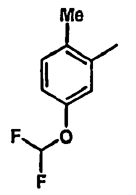 | 0 | 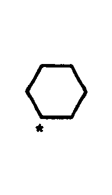 | | N | CH | C(O)OMe | 119 |
| 17 | 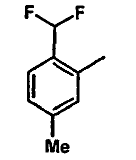 | 0 | 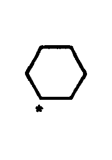 | | N | CH | C(O)OMe | 124 |
| 18 | 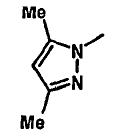 | 0 | 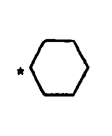 | | N | CH | C(O)OMe | 158 |
| 19 | 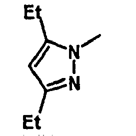 | 0 | 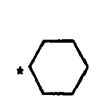 | | N | CH | C(O)OMe | 128 |
| 20 | 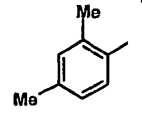 | 0 | 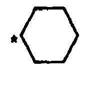 | | N | GN | C(O)OMe | 118 |
| 21 | 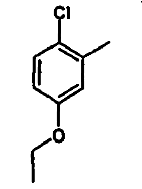 | 0 | 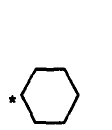 | | N | N | C(O)OMe | 124 |
| 22 | 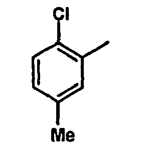 | 1 | CH(Me)₂ énantiomère (S) | H | N | CH | C(O)OMe | 166 |
| 23 | 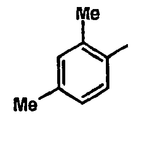 | 1 | C(Me)₃ énantiomère (S) | H | N | CH | C(O)OMe | 129 |
| 24 | 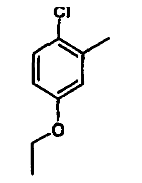 | 1 | C(Me)₃ énantiomère (S) | H | N | N | C(O)OMe | 112 |
| 25 | 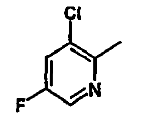 | 1 | C(Me)₃ énantiomère (S) | H | N | CH | C(O)OMe | 130 |
| 26 | 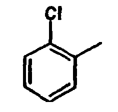 | 0 | 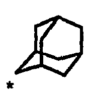 | | N | CH | C(O)OMe | 135 |
| 27 | 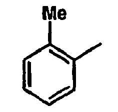 | 1 | C(Me)₃ énantiomère (S) | H | N | CH | C(O)O(CH₂)₂O CH₃ | 85 |
| 28 | 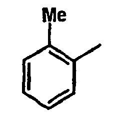 | 1 | C(Me)₃ énantiomère (S) | H | N | CH | C(O)OMe | 93 |
| 29 | 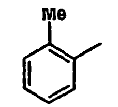 | 1 | C(Me)₃ énantiomère (S) | H | N | CH | C(O)OEt | 90 |
| 30 | 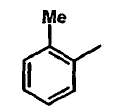 | 1 | C(Me)₃ énantiomère (S) | H | N | CH | C(O)OiPr | 190 |
| 31 Ex 2 | 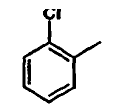 | 1 | C(Me)₃ énantiomère (S) | H | N | CH | C(O)OMe | 126 |
| 32 | 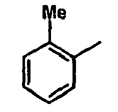 | 1 | C(Me)₃ énantiomère (S) | H | N | CH | C(O)NH₂ | 152 |
| 33 | 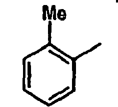 | 1 | C(Me)₃ énantiomère (S) | H | N | CH | C(O)NHMe | 152 |
| 34 Ex6 | 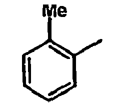 | 1 | C(Me)₃ énantiomère (S) | H | N | CH | C(O)NHSO₂Me | 138 |
| 35 | 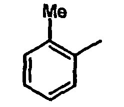 | 1 | C(Me)₃ énantiomère (S) | H | N | CH | C(O)NHCH₂C F₃ | 142 |
| 36 | 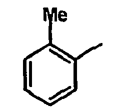 | 1 | C(Me)₃ énantiomère (S) | H | N | CH | 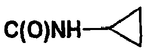 | 151 |
| 37 Ex7 | 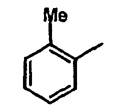 | 1 | C(Me)₃ énantiomère (S) | H | N | CH | CH₂OH | 93 |
| 38 | 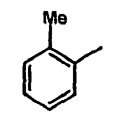 | 1 | C(Me)₃ énantiomère (S) | H | N | CH | CH₂OMe | 108 |
| 39 | 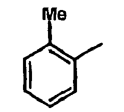 | 1 | C(Me)₃ énantiomère (S) | H | N | CH | CN | 140 |

**TABLEAU 2**

| | | | |
|---|---|---|---|
| 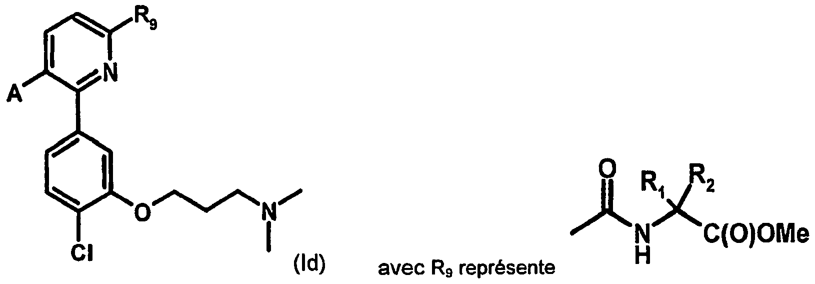 | | | |

| N° | A | R₉ | Point de fusion (°C) |
|---|---|---|---|
| 40 | 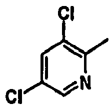 | 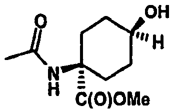 | 202 |
| 41 | 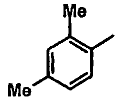 | 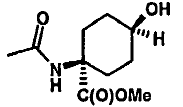 | 253 |
| 42 Ex 5 | 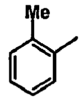 | 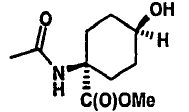 | 244 |
| 43 | 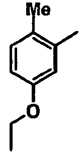 | 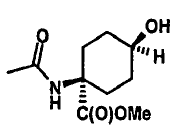 | 160 |
| 44 | 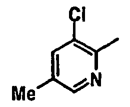 | 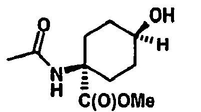 | 178 |

Les composés selon l'invention ont fait l'objet d'essais pharmacologiques en vue de déterminer des propriétés des composés de l'invention, avec en particulier un test *in vitro* de mobilisation calcique intracellulaire (test FlipR) mettant en jeu des antagonistes à l'Urotensine II (les composés de la présente invention) du récepteur GPR14 humain.

Ce test est décrit ci-dessous :

### 1. Protocole FlipR (Fluorometric Imaging Plate Reader)

### 1.1 Objectif

L'objectif est de mesurer l'activation du Récepteur GPR14 par l'Urotensine II humaine.

### 1.2. Principe du Test

Le GPR14 est un récepteur à 7 domaines transmenbranaires couplé Gq. Son activation par un ligand spécifique provoque une augmentation de Ca²⁺ dans la cellule via la voie PLC (Phospholipase C), IP3 (Inositol-1,4,5-triphosphate) DAG (Diacylclycerol). L'augmentation de Ca²⁺ dans la cellule est mesurée à l'aide de la sonde perméante Fluo4AM (sonde mono excitation, mono émission) qui se lie au Ca²⁺ libre et émet à 520 nm. La sonde libre est non fluorescente en absence de Ca²⁺.

### 1.3.Protocole

### Plan de l'expérience

1) Ensemencement des cellules à J-1 (Jour -1) ou J-2
2) Incorporation/charge (J0) de la sonde (1 h)
3) Ajout des produits au FlipR et mesure
4) Ajout du ligand au FlipR et mesure en présence des produits
5) Traitement et export des données

### Cellules CHOGPR14

Les cellules sont cultivées en milieu complet en Flask T225. Pour les expériences, les cellules sont repiquées dans 200µl de milieu de culture en plaques 96 puits noires à fond transparent à raison de 60.000 cellules/puits pour une utilisation à J+1 ou 40.000 cellules /puits pour une utilisation à J+2.

### Incorporation du Fluo 4 M

Le Fluo-4AM est préparé à 20 mM puis aliquoté (50 µl) et conservé à -20°C à l'abri de la lumière. Une solution d'acide pluronique à 200mg/ml dans le DMSO est également préparée (elle se conserve une semaine à T.A. à l'abri de la lumière).

Les cellules sont chargées avec le mélange Fluo-4AM + acide pluronique (aliquot 50µl + 50µl d'acide pluronique) dilués au1/100 dans le tampon de mesure.

Après un lavage des puits par 150 µl de tampon de mesure (cf annexe), les cellules sont ensuite chargées de la manière suivante :
- répartition de 100µl de tampon de mesure dans chaque puits
- addition de 10µl du mélange Fluo-4AM + acide pluronique dilué au 1/100.

Les cellules sont incubées 1 h à 37°C à l'abri de la lumière, dans un incubateur en présence de 5% de CO₂.

Les cellules sont ensuite lavées 3 fois par 150 µl de tampon de mesure pour éliminer l'excès de sonde. Un volume de 150 µl de tampon est ajouté dans chaque puits en fin de lavage.

Après une incubation des plaques 20 min à T.A. à l'abri de la lumière, celles-ci sont placées dans le FlipR en vue de la mesure de la fluorescence.

Le niveau d'incorporation de base du Fluo-4 est vérifié pour chaque plaque (sd < 10%) avant la première injection.

Après une stabilisation du signal de base, les composés inhibiteurs du GPR14 sont injectés par le FlipR sous un volume de 50 µl à partir d'une plaque de dilution effectuée au Biomek 2000 en tampon de mesure. L'Urotensine II (3 nM final, concentration égale à la CE₅₀) est rajoutée sous un volume de 50 µl par le FlipR sur les cellules à partir d'une plaque stock à 15 nM diluée dans le tampon de mesure. L'enregistrement des données s'effectue en continu pendant toute la durée de l'expérience.

### 1.4. Analyse des données

Pour chaque plaque, la fluorescence de base avant l'injection des composés est normalisée par la fonction « spatial uniformity correction » du FlipR. Les valeurs de fluorescence mesurées juste avant l'injection de l'Urotensine II (min) ainsi que celles de la fluorescence mesurée au pic de l'effet de l'Urotensine II (max) sont exportées sous Excel. Dans chaque plaque, une série de puits est traitée par l'Urotensine seule en l'absence de composé inhibiteur. Les valeurs de fluorescence min et max pour ces puits sont moyennées et permettent de définir le 100 % de l'effet de l'Urotensine II.

Les pourcentages d'inhibition calculés pour chaque concentration d'inhibiteur sont calculés de la manière suivante :
- pour chaque puits Uro II (Urotensine II) + inhibiteur, calcul de la valeur delta produit = max - min
- pour les puits Uro II seule, calcul de la valeur delta Uroll (moyenne max - moyenne min)

Le pourcentage d'inhibition pour chaque concentration de produit est calculé comme décrit ci-dessous :
Inhibition (%) = 100 x (delta Uro II - delta produit)/delta Uroll

### 1.5. Annexe

Composition du tampon de mesure (dans l'eau déminéralisée à préparer extemporanément)

| | Qsp 500 mL | QSP 1 L | QSP 2 L |
|---|---|---|---|
| HBSS | 50 mL | 100 mL | 200 mL |
| MgSO₄ 19,72 g/L | 5 mL | 10 mL | 20 mL |
| Hépès | 2,38 g | 4,76 g | 952g |
| Na₂CO₃ 35 g/L | 5 mL | 10 mL | 20 mL |
| CaCl₂ 14,7 g/L | 5 mL | 10 mL | 20 mL |
| BSA 10% | 50 mL | 100 mL | 200 mL |

| | | | |
|---|---|---|---|
| HBSS = Hanks' Balanced Salt Solution BSA = Bovine Serum Albumine | | | |

Les différentes solutions salines peuvent être conservées en stock 2 mois à 4°C.

Ajuster le volume H₂O et ajouter le probenecid dissout dans la soude 1 N

| | | | |
|---|---|---|---|
| + Probenecid | 0,355 g dans 5 mL de NaOH 1N | 0,71 g dans 10 mL de NaOH 1 N | 1,42 g dans 20 mL NaOH 1 N |

Vérifier le PH 7.4.

### 1.6. Matériel

Urotensine II humaine (Bachem H-4768)
Fluo-4AM (Molecular Probes (F14202 5 x 1mg)
Probenecid (Sigma P8761 100g)
Acide Pluronique (Molecular Probes P6867)
HBSS 10x (Gibco 14185-045)
HEPES (acide) (Sigma H3375)
Sodium carbonate (Sigma S7795) Na₂CO₃
Magnesium sulfate (Sigma M7774) MgSO₄
Calcium chlorure (Sigma C5080) CaCl₂
Pointes noires (Molecular Devices 9000-0549)
Plaques noires 96 puits (Beckton Dickinson 356640)
DMSO (Sigma D 5879)

### 1.7. Résultats

Les composés testés ont un IC50 dans le test FlipR inférieur à 10000 nM. Certains de ces composés ont un IC50 dans le test FlipR inférieur à 1000 nM, voire inférieur à 200 nM. A titre d'exemples, les composés n° 1, 2, 4, 5, 7, 9, 11, 13, 18, 20, 24, 26, 29, 33, 36, 42, 44, du tableau présentent des CI50 de 1600, 1700, 630, 540, 330, 1400, 566, 846, 1080, 116, 102, 170, 253, 450, 450, 95, 660 nM, respectivement.

Les composés selon l'invention peuvent donc être utilisés pour la préparation de médicaments, en particulier de médicaments inhibiteurs des récepteurs de l'Urotensine II. Ainsi, selon un autre de ses aspects, l'invention a pour objet des médicaments qui comprennent un composé de formule (I), ou un sel d'addition de ce dernier à un acide pharmaceutiquement acceptable du composé de formule (I) ou encore un énantiomère, un diastéréoisomère ou un mélange racémique de ce composé,.

Ces médicaments trouvent leur emploi en thérapeutique, notamment dans le traitement et/ou la prévention de l'insuffisance cardiaque congestive, de l'ischémie cardiaque, de l'infarctus du myocarde, de l'hypertrophie et la fibrose cardiaque, des maladies coronaires et de l'athérosclérose, de l'hypertension artérielle systémique, de l'hypertension pulmonaire, de l'hypertension portale, de la fibrose hépatique, de la resténose post angioplastie, des insuffisances rénales et plus particulièrement aïgue et chronique d'origine diabétique et/ou hypertensive, du diabète, de l'inflammation en général, de la fibrose en général et des anévrismes.

Ces médicaments trouvent également leur emploi en thérapeutique, dans le traitement et/ou la prévention des désordres du système nerveux central, incluant notamment les les maladies neurodégénératives, les accidents vasculaires cérébraux, le stress, l'anxiété, l'agressivité, la dépression, la schizophrénie ou encore des maladies du sommeil.

Des médicaments comprenant des composés antagonistes de l'Urotensine II tels que les composés selon l'invention trouvent également leur emploi en thérapeutique, dans le traitement et/ou la prévention des vomissements.

Ces médicaments trouvent également leur emploi en thérapeutique dans le traitement de certains cancers.

Ces médicaments trouvent également leur emploi en thérapeutique, dans le traitement et/ou la prévention de l'asthme et des maladies respiratoires.

Il est à noter que les composés selon l'invention qui ont un groupe R3 correspondant à une fonction ester, après absorption par voie orale, peuvent subir une hydrolyse enzymatique pour libérer l'acide correspondant et ainsi être utiles en tant que prodrogues.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, un énantiomère, un diastéréoisomère ou un mélange racémique de ce composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables, ou encore un énantiomère, un diastéréoisomère ou un mélange racémique de ce composé.

## Revendications

1. Composé répondant à la formule (I) 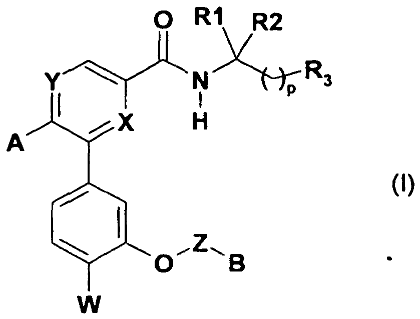 dans laquelle:
X et Y représentent indépendamment l'un de l'autre un atome d'azote ou un chaînon -CR4-, où R4 représente un atome d'hydrogène ou un groupe (C1-C4) alkyle ou alcoxy ;
A représente un groupe aryle ou hétéroaryle, les dits groupes aryle ou hétéroaryle étant éventuellement substitués par un ou plusieurs groupes choisis parmi un atome d'halogène, un groupe hydroxy, (C1-C4) alkyle, (C3-C5)cycloalkyle, (C1-C4) alcoxy éventuellement substitué par un groupe (C1-C4)alcoxy, halogénoalkyle, halogénoalcoxy, un groupe nitrile ;
W représente un atome d'halogène ou un groupe halogénoalkyle ;
Z représente un groupe (C1-C4)alkylène éventuellement substitué par un ou plusieurs groupes choisis parmi un atome d'halogène et les groupes (C1-C4)alkyle, hydroxy et (C1-C4)alcoxy ;
B représente un groupe -NR4R5, où R4 et R5 représentent indépendamment l'un de l'autre un groupe (C1-C4)alkyle ;
R1 et R2 représentent :
• soit, R1 représente un atome d'hydrogène et R2 représente un groupe (C1-C4)alkyle,
• soit R1 et R2 forment ensemble, avec l'atome de carbone auquel ils sont rattachés, un système mono ou polycyclique choisi parmi : un groupe (C3-C8)cycloalkyle, un groupe bicyclique ponté ou un groupe tetracyclique ponté, ledit système pouvant être substitué par un ou plusieurs groupes hydroxy ;
R3 représente :
• soit un groupe C(O)R5 avec R5 représente un groupe (C1-C4)alcoxy éventuellement substitué par un groupe (C1-C4)alcoxy ou un groupe NR6R7 avec R6 et R7 indépendamment l'un de l'autre représentent un atome d'hydrogène, un groupe (C1-C4)alkyle, (C3-C5)cycloalkyle, (C1-C4)alkylesulfonyle, halogénoalkyle,
• soit un groupe -CH₂XR8 avec :
- X représente un atome d'oxygène et R8 représente un atome d'hydrogène ou un groupe (C1-C4)alkyle,
- ou X représente un groupe NH et R8 représente un groupe (C1-C4)alkylecarbonyle, (C1-C4)alkylecarboxyle ou (C1-C4)alkylesulfonyle,
• soit un groupe nitrile (CN) ;
p représente un nombre entier égal à 0 ou 1,
à l'état de base ou de sel d'addition à un acide ou à une base ainsi que leurs énantiomères et diastéréoisomères, y compris leurs mélanges racémiques.

2. Composé de formule (I) selon la revendication 1, **caractérisé en ce que**
X et Y représentent indépendamment l'un de l'autre un atome d'azote ou un chaînon -CR4-, où R4 représente un atome d'hydrogène ;
A représente un groupe aryle ou hétéroaryle, les dits groupes aryle ou hétéroaryle étant éventuellement substitués par un ou plusieurs groupes choisis parmi un atome d'halogène, un groupe hydroxy, (C1-C4) alkyle, (C3-C5)cycloalkyle, (C1-C4) alcoxy éventuellement substitué par un groupe (C1-C4) alcoxy, halogénoalkyle, halogénoalcoxy,
W représente un atome d'halogène;
Z représente un groupe (C1-C4)alkylène ;
B représente un groupe -NR4R5, où R4 et R5 représentent indépendamment l'un de l'autre un groupe (C1-C4)alkyle,
R1 et R2 représentent :
• soit, R1 représente un atome d'hydrogène et R2 représente un groupe (C1-C4)alkyle,
• soit R1 et R2 forment ensemble, avec l'atome de carbone auquel ils sont rattachés, un système mono ou polycyclique choisi parmi un groupe (C3-C8)cycloalkyle et un groupe tetracyclique ponté, ledit système pouvant être substitué par un ou plusieurs groupes hydroxy ;
R3 représente :
• soit un groupe C(O)R5 avec R5 représente un groupe (C1-C4)alcoxy éventuellement substitué par un groupe (C1-C4)alcoxy ou un groupe NR6R7 avec R6 et R7 indépendamment l'un de l'autre représentent un atome d'hydrogène, un groupe (C1-C4)alkyle, (C3-C5)cycloalkyle, (C1-C4)alkylesulfonyle, halogénoalkyle,
• soit un groupe -CH₂XR8 avec X représente un atome d'oxygène et R8 représente un atome d'hydrogène ou un groupe (C1-C4)alkyle,
• soit un groupe nitrile (CN) ;
p représente un nombre entier égal à 0 ou 1,
à l'état de base ou de sel d'addition à un acide ou à une base ainsi que leurs énantiomères et diastéréoisomères, y compris leurs mélanges racémiques.

3. Composé de formule (I) selon la revendication 1, **caractérisé en ce que** :
X représente un atome d'azote et Y représente un atome d'azote ou un chaînon - CR4-, où R4 représente un atome d'hydrogène ;
A représente un groupe aryle étant éventuellement substitué par un ou plusieurs groupes choisis parmi un atome d'halogène, (C1-C4)alkyle, (C3-C5)cycloalkyle, (C1-C4)alcoxy, halogénoalcoxy ou représente un groupe hétéroaryle étant éventuellement substitué par un ou plusieurs groupes choisis parmi un atome d'halogène, (C1-C4)alkyle;
W représente un atome d'halogène,
Z représente un groupe (C1-C4)alkylène ;
B représente un groupe -NR4R5, où R4 et R5 représentent indépendamment l'un de l'autre un groupe (C1-C4)alkyle,
R1 et R2 représentent :
• soit, R1 représente un atome d'hydrogène et R2 représente un groupe (C1-C4)alkyle,
• soit R1 et R2 forment ensemble, avec l'atome de carbone auquel ils sont rattachés, un groupe (C3-C8)cycloalkyle éventuellement susbtitué par un ou plusieurs groupes hydroxy ou un groupe adamantyle ;
R3 représente :
• soit un groupe C(O)R5 avec R5 représente un groupe (C1-C4)alcoxy éventuellement substitué par un groupe (C1-C4)alcoxy ou un groupe NR6R7 avec R6 représente un atome d'hydrogène et R7 représente un atome d'hydrogène, un groupe (C1-C4)alkyle, (C3-C5)cycloalkyle, (C1-C4)alkylesulfonyle, halogénoalkyle,
• soit un groupe -CH₂XRB avec X représente un atome d'oxygène et R8 représente un atome d'hydrogène ou un groupe (C1-C4)alkyle ;
p représente un nombre entier égal à 0 ou 1,
à l'état de base ou de sel d'addition à un acide ou à une base ainsi que leurs énantiomères et diastéréoisomères, y compris leurs mélanges racémiques.

4. Composé de formule (I) selon la revendication 1, **caractérisé en ce qu'**il est un chlorhydrate.

5. Composé de formule (I) selon la revendication 1, **caractérisé en ce qu'**il est choisi parmi les composés suivants :
1-{[(3,5-dichloro-2'-{4-chloro-3-[3-(diméthylamino)propoxy]phényl}-2,3'-bipyridin-6'-yl)carbonyl]amino}cyclohexanecarboxylate de méthyle ;
1-({[6-{4-chloro-3-[3-(diméthylamino)propoxy]phényl}-5-(4-hydroxy-2-méthylphényl)pyridin-2-yl]carbonyl}amino)cyclohéxanecarboxylate de méthyle ;
1-({[6-{4-chloro-3-[3-(diméthylamino)propoxy]phényl}-5-(5-hydroxy-2-méthylphényl)pyridin-2-yl]carbonyl}amino)cyclohexanecarboxylate de méthyle ;
1-({[6-{4-chloro-3-[3-(diméthylamino)propoxy]phényl}-5-(2-méthylphényl)pyridin-2-yl]carbonyl}amino)cyclohexanecarboxylate de méthyle ;
1-{[(3-chloro-2'-{4-chloro-3-[3-(diméthylamino)propoxy]phényl}-5-méthyl-2,3'-bipyridin-6'-yl)carbonyl]amino}cyclohexanecarboxylate de méthyle ;
1-{[(6-{4-chloro-3-[3-(diméthylamino)propoxy]phényl}-5-[5-(2-méthoxyéthoxy)-2-méthylphényl]pyridin-2-yl)carbonyl]amino}cyclohexanecarboxylate de méthyle ;
1-({[6-{4-chloro-3-[3-(diméthylamino)propoxy]phényl}-5-(5-cyclopropyl-2-méthylphényl)pyridin-2-yl]carbonyl}amino)cyclohexanecarboxylate de méthyle ;
1-({[6-{4-chloro-3-[3-(diméthylamino)propoxy]phényl}-5-(5-isopropoxy-2-méthylphényl)pyridin-2-yl]carbonyl}amino)cyclohexanecarboxylate de méthyle ;
1-({[6-{4-chloro-3-[3-(diméthylamino)propoxy]phényl}-5-(2-chloro-5-propoxyphényl)pyridin-2-yl]carbonyl}amino)cyclohexanecarboxylate de méthyle ;
1-({[6-{4-chloro-3-[3-(diméthylamino)propoxy]phényl}-5-(5-éthoxy-2-méthylphényl)pyridin-2-yl]carbonyl}amino)cyclohexanecarboxylate de méthyle ;
1-({[6-{4-chloro-3-[3-(diméthylamino)propoxy]phényl}-5-(6-méthyl-1,3-benzodioxol-5-yl)pyridin-2-yl]carbonyl}amino)cyclohexanecarboxylate de méthyle ;
1-{[(3-chloro-2'-{4-chloro-3-[3-(diméthylamino)propoxy]phényl}-5-fluoro-2,3'-bipyridin-6'-yl)carbonyl]amino}cyclohéxanecarboxylate de méthyle ;
1-{[(2'-{4-chloro-3-[3-(diméthylamino)propoxy]phényl}-3,5-diméthyl-2,3'-bipyridin-6'-yl)carbonyl]amino}cyclohexanecarboxylate de méthyle ;
1-({[6-{4-chloro-3-[3-(diméthylamino)propoxy]phényl}-5-(3-hydroxy-2-méthylphényl)pyridin-2-yl]carbonyl}amino)cyclohexanecarboxylate de méthyle ;
1-{[(2-{4-chloro-3-[3-(diméthylamino)propoxy]phényl}-2'-méthyl-3,3'-bipyridin-6-yl)carbonyl]amino}cyclohexanecarboxylate de méthyle ;
1-{[(6-{4-chloro-3-[3-(diméthylamino)propoxy]phényl}-5-[5-(difluorométhoxy)-2-méthylphényl]pyridin-2-yl)carbonyl]amino}cyclohexanecarboxylate de méthyle ;
1-{[(6-{4-chloro-3-[3-(diméthylamino)propoxy]phényl}-5-[2-(difluorométhyl)-5-méthylphényl]pyridin-2-yl)carbonyl]amino}cyclohexanecarboxylate de méthyle ;
1-({[6-{4-chloro-3-[3-(diméthylamino)propoxy]phényl}-5-(3,5-diméthyl-1*H*-pyrazol-1-yl)pyridin-2-yl]carbonyl}amino)cyclohéxanecarboxylate de méthyle ;
1-({[6-{4-chloro-3-[3-(diméthylamino)propoxy]phényl}-5-(3,5-diéthyl-1*H*-pyrazol-1-yl)pyridin-2-yl]carbonyl}amino)cyclohexanecarboxylate de méthyle ;
1-({[6-{4-chloro-3-[3-(diméthylamino)propoxy]phényl}-5-(2,4-diméthylphényl)pyrazin-2-yl]carbonyl}amino)cyclohexanecarboxylate de méthyle ;
1-({[6-{4-chloro-3-[3-(diméthylamino)propoxy]phényl}-5-(2-chloro-5-éthoxyphényl)pyrazin-2-yl]carbonyl}amino)cyclohexanecarboxylate de méthyle ;
(3*S*)-3-({[6-{4-chloro-3-[3-(diméthylamino)propoxy]phényl}-5-(2-chloro-5-méthylphényl)pyridin-2-yl]carbonyl}amino)-4-méthylpentanoate de méthyle ;
(3*S*)-3-({[6-{4-chloro-3-[3-(diméthylamino)propoxy]phényl}-5-(2,4-diméthylphényl)pyridin-2-yl]carbonyl}amino)-4,4-diméthylpentanoate de méthyle ;
(3*S*)-3-({[6-{4-chloro-3-[3-(diméthylamino)propoxy]phényl}-5-(2-chloro-5-éthoxyphényl)pyrazin-2-yl]carbonyl}amino)-4,4-diméthylpentanoate de méthyle ;
(3*S*)-3-{[(3-chloro-2'-{4-chloro-3-[3-(diméthylamino)propoxy]phényl}-5-fluoro-2,3'-bipyridin-6'-yl)carbonyl]amino}-4,4-diméthylpentanoate de méthyle ;
2-({[6-{4-chloro-3-[3-(diméthylamino)propoxy]phényl}-5-(2-chlorophényl)pyridin-2-yl]carbonyl}amino)adamantane-2-carboxylate de méthyle ;
(3*S*)-3-({[6-{4-chloro-3-[3-(diméthylamino)propoxy]phényl}-5-(2-méthylphényl)pyridin-2-yl]carbonyl}amino)-4,4-diméthylpentanoate de 2-methoxyéthyle ;
(3*S*)-3-({[6-{4-chloro-3-[3-(diméthylamino)propoxy]phényl}-5-(2-méthylphényl)pyridin-2-yl]carbonyl}amino)-4,4-diméthylpentanoate de méthyle ;
(3*S*)-3-({[6-{4-chloro-3-[3-(diméthylamino)propoxy]phényl}-5-(2-méthylphényl)pyridin-2-yl]carbonyl}amino)-4,4-diméthylpentanoate d'éthyle ;
(3*S*)-3-({[6-{4-chloro-3-[3-(diméthylamino)propoxy]phényl}-5-(2-méthylphényl)pyridin-2-yl]carbonyl}amino)-4,4-diméthylpentanoate d'isopropyle ;
(3*S*)-3-({[6-{4-chloro-3-[3-(diméthylamino)propoxy]phényl}-5-(2-chlorophényl)pyridin-2-yl]carbonyl}amino)-4,4-diméthylpentanoate de méthyle ;
*N*-[(1*S*)-1-(2-amino-2-oxoéthyl)-2,2-diméthylpropyl]-6-{4-chloro-3-[3-(diméthylamino)propoxy]phényl}-5-(2-méthylphényl)pyridine-2-carboxamide ;
6-{4-chloro-3-[3-(diméthylamino)propoxylphényl}-*N*-{(1S)-2,2-diméthyl-1-[2-(méthylamino)-2-oxoéthyl]propyl}-5-(2-méthylphényl)pyridine-2-carboxamide ;
6-{4-chloro-3-[3-(diméthylamino)propoxy]phényl}-*N*-[(1S)-2,2-diméthyl-1-{2-[(méthylsulfonyl)amino]-2-oxoéthyl}propyl]-5-(2-méthylphényl)pyridine-2-carboxamide ;
6-{4-chloro-3-[3-(diméthylamino)propoxy]phényl}-*N*-[(1*S*)-1-{2-[(1,1,1-trifluoroéthyl)amino]-2-oxoéthyl}-2,2-diméthylpropyl]-5-(2-méthylphényl)pyridine-2-carboxamide ;
6-{4-chloro-3-[3-(diméthylamino)propoxy]phényl}-*N*-[(1*S*)-1-{2-[cyclopropyl(méthyl)amino]-2-oxoéthyl}-2,2-diméthylpropyl]-5-(2-méthylphényl)pyridine-2-carboxamide ;
6-{4-chloro-3-[3-(diméthylamino)propoxy]phényl}-*N*-[(1S)-1-(2-hydroxyéthyl)-2,2-diméthylpropyl]-5-(2-méthylphényl)pyridine-2-carboxamide ;
6-{4-chloro-3-[3-(diméthylamino)propoxy]phényl}-*N*-[(1*S*)-1-(2-méthoxyéthyl)-2,2-diméthylpropyl]-5-(2-méthylphényl)pyridine-2-carboxamide ;
6-{4-chloro-3-[3-(diméthylamino)propoxy]phényl}-*N*-[(2*S*)-1-cyano-3,3-diméthylbutan-2-yl]-5-(2-méthylphényl)pyridine-2-carboxamide ;
*cis*-1-{[{3,5-dichloro-2'-{4-chloro-3-[3-(diméthylamino)propoxy]phényl}-2,3'- bipyridin- 6'-yl)carbonyl]amino}-4-hydroxycyclohexanecarboxylate de méthyle ;
*cis*-1-({[6-{4-chloro-3-[3-(diméthylamino)propoxy]phényl}-5-(2,4-diméthylphényl)pyridin-2-yl]carbonyl}amino)-4-hydroxycyclohexanecarboxylate de méthyle ;
*cis*-1-({[6-{4-chloro-3-[3-(diméthylamino)propoxy]phényl}-5-(2-méthylphényl)pyridin-2-yl]carbonyl}amino)-4-hydroxycyclohexanecarboxylate de méthyle ;
*cis*-1-({[6-{4-chloro-3-[3-(diméthylamino)propoxy]phényl}-5-(5-éthoxy-2-méthylphényl)pyridin-2-yl]carbonyl}amino)-4-hydroxycyclohexanecarboxylate de méthyle ;
*cis*-1-{[(3-chloro-2'-{4-chloro-3-[3-(diméthylamino)propoxy]phényl}-5-méthyl-2,3'-bipyridin-6'-yl)carbonyl]amino}-4-hydroxycyclohexanecarboxylate de méthyle.

6. Composé de formule (I) selon la revendication 1, **caractérisé en ce qu'**il est choisi parmi les composés suivants :
1-{[(3,5-dichloro-2'-{4-chloro-3-[3-(diméthylamino)propoxy]phényl}-2,3'-bipyridin-6'-yl)carbonyl]amino}cyclohexanecarboxylate de méthyle ;
1-({[6-{4-chloro-3-[3-(diméthylamino)propoxy]phényl}-5-(2-méthylphényl)pyridin-2-yl]carbonyl}amino)cyclohexanecarboxylate de méthyle ;
1-{[(3-chloro-2'-{4-chloro-3-[3-(diméthylamino)propoxy]phényl}-5-méthyl-2,3'-bipyridin-6'-yl)carbonyl]amino}cyclohexanecarboxylate de méthyle ;
(3*S*)-3-({[6-{4-chloro-3-[3-(diméthylamino)propoxy]phényl}-5-(2-chlorophényl)pyridin-2-yl]carbonyl}amino)-4,4-diméthylpentanoate de méthyle ;
6-{4-chloro-3-[3-(diméthylamino)propoxy]phényl}-*N*-[(1S)-2,2-diméthyl-1-{2-[(méthylsulfonyl)amino]-2-oxoéthyl}propyl]-5-(2-méthylphényl)pyridine-2-carboxamide ;
6-{4-chloro-3-[3-(diméthylamino)propoxy]phényl}-*N*-[(1*S*)-1-(2-hydroxyéthyl)-2,2-diméthylpropyl]-5-(2-méthylphényl)pyridine-2-carboxamide ;
cis-1-({[6-{4-chloro-3-[3-(diméthylamino)propoxy]phényl}-5-(2-méthylphényl)pyridin-2-yl]carbonyl}amino)-4-hydroxycyclohexanecarboxylate de méthyle .

7. Procédé de préparation d'un composé de formule (I) selon l'une quelconque des revendications 1 à 6, **caractérisé en ce que** l'on fait réagir un composé de formule (II) : 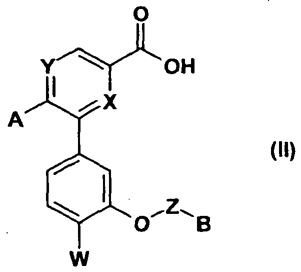 dans laquelle X, Y, Z, W, A et B sont tels que définis dans la revendication 1, avec un composé de formule (III) : 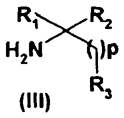 dans lequel R1, R2 et R3 sont tels que définis dans la revendication 1.

8. Procédé de préparation d'un composé de formule (I) selon l'une quelconque des revendications 1 à 6, **caractérisé en ce que** l'on fait réagir un composé de formule (IV) : 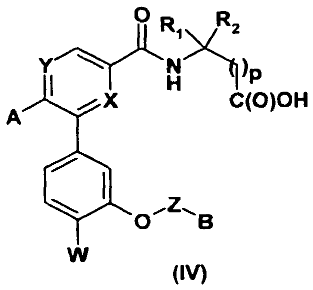 dans laquelle X, Y, Z, W, A et B sont tels que définis dans la revendication 1, avec un composé de formule R5H avec R5 tel que défini dans la revendication 1.

9. Procédé de préparation d'un composé de formule (I) selon l'une quelconque des revendications 1 à 6, **caractérisé en ce que** l'on fait réagir un composé de formule (IV) : 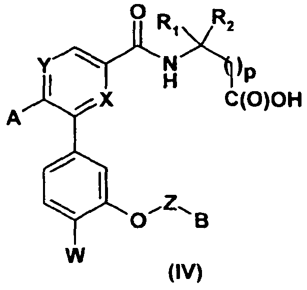 avec un agent réducteur.

10. Médicament, **caractérisé en ce qu'**il comprend un composé de formule (I) selon l'une quelconque des revendications 1 à 6, ou un sel d'addition de ce composé à un acide ou à une base pharmaceutiquement acceptable ou encore un énantiomère, un diastéréoisomère ou un mélange racémique de ce composé.

11. Composition pharmaceutique, **caractérisé en ce qu'**il comprend un composé de formule (I) selon l'une quelconque des revendications 1 à 6, ou un sel d'addition de ce composé à un acide ou à une base pharmaceutiquement acceptable, un énantiomère, un diastéréoisomère ou un mélange racémique de ce composé, ainsi qu'au moins un excipient pharmaceutiquement acceptable.

12. Utilisation d'un composé de formule (I) selon l'une quelconque des revendications 1 à 6 pour la préparation d'un médicament destiné au traitement et/ou à la prévention de l'insuffisance cardiaque congestive, de l'ischémie cardiaque, de l'infarctus du myocarde, de l'hypertrophie et la fibrose cardiaque, des maladies coronaires et de l'athérosclérose, de l'hypertension artérielle systémique et pulmonaire, de l'hypertension portale, de la fibrose hépatique de la resténose post-angioplastie, des insuffisances rénales aigüe et chronique d'origine diabétique et/ou hypertensive, du diabète, d'inflammation, de fibroses et des anévrismes, des désordres du système nerveux central, incluant les maladies neurodégénératives, les accidents vasculaires cérébraux, le stress, l'anxiété, l'agressivité, la dépression, la schizophrénie ou encore des maladies du sommeil et de cancers ou de maladies respiratoires, de l'asthme.

13. Utilisation d'un composé de formule (I) selon l'une quelconque des revendications 1 à 6 pour la préparation d'un médicament destiné au traitement et/ou à la prévention des insuffisances rénales aigüe et chronique d'origine diabétique.

14. Utilisation d'un composé de formule (I) selon l'une quelconque des revendications 1 à 6 pour la préparation d'un médicament destiné au traitement et/ou à la prévention du diabète.

15. Utilisation d'un composé de formule (I) selon l'une quelconque des revendications 1 à 6 pour la préparation d'un médicament destiné au traitement et/ou à la prévention des vomissements.

## Claims

1. Compound corresponding to formula (I) 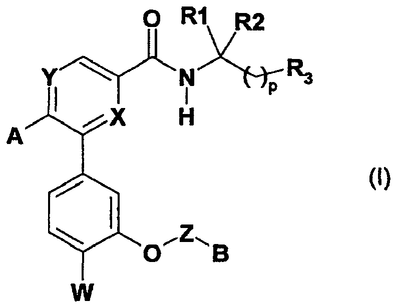 in which:
X and Y represent, independently of each other, a nitrogen atom or a chain -CR4-, in which R4 represents a hydrogen atom or a (C1-C4) alkyl or alkoxy group;
A represents an aryl or heteroaryl group, said aryl or heteroaryl groups being optionally substituted with one or more groups chosen from a halogen atom, a hydroxyl group, (C1-C4) alkyl, (C3-C5) cycloalkyl, (C1-C4) alkoxy optionally substituted with a (C1-C4) alkoxy, haloalkyl or haloalkoxy group, or a nitrile group;
W represents a halogen atom or a haloalkyl group;
Z represents a (C1-C4) alkylene group optionally substituted with one or more groups chosen from a halogen atom and (C1-C4) alkyl, hydroxyl and (C1-C4) alkoxy groups;
B represents a group -NR4R5, in which R4 and R5 represent, independently of each other, a (C1-C4) alkyl group;
R1 and R2 represent:
• either, R1 represents a hydrogen atom and R2 represents a (C1-C4) alkyl group,
• or R1 and R2 form, together with the carbon atom to which they are attached, a monocyclic or polycyclic system chosen from: a (C3-C8) cycloalkyl group, a bridged bicyclic group or a bridged tetracyclic group, said system possibly being substituted with one or more hydroxyl groups;
R3 represents:
• either a group C(O)R5 with R5 representing a (C1-C4) alkoxy group optionally substituted with a (C1-C4) alkoxy group or a group NR6R7 with R6 and R7, independently of each other, representing a hydrogen atom or a (C1-C4) alkyl, (C3-C5) cycloalkyl, (C1-C4) alkylsulfonyl or haloalkyl group,
• or a group CH₂XR8 in which:
- X represents an oxygen atom and R8 represents a hydrogen atom or a (C1-C4) alkyl group,
- or X represents an NH group and R8 represents a (C1-C4) alkylcarbonyl, (C1-C4) alkylcarboxyl or (C1-C4)alkylsulfonyl group,
• or a nitrile group (CN);
p represents an integer equal to 0 or 1,
in the form of base or of addition salt with an acid or a base, and also the enantiomers and diastereoisomers thereof, including racemic mixtures thereof.

2. Compound of formula (I) according to Claim 1, **characterized in that**
X and Y represent, independently of each other, a nitrogen atom or a chain -CR4-, in which R4 represents a hydrogen atom;
A represents an aryl or heteroaryl group, said aryl and heteroaryl groups being optionally substituted with one or more groups chosen from a halogen atom, a hydroxyl group, (C1-C4) alkyl, (C3-C5) cycloalkyl, (C1-C4) alkoxy optionally substituted with a (C1-C4) alkoxy, haloalkyl or haloalkoxy group;
W represents a halogen atom;
Z represents a (C1-C4) alkylene group;
B represents a group -NR4R5, in which R4 and R5 represent, independently of each other, a (C1-C4) alkyl group,
R1 and R2 represent:
• either, R1 represents a hydrogen atom and R2 represents a (C1-C4) alkyl group,
• or R1 and R2 form, together with the carbon atom to which they are attached, a monocyclic or polycyclic system chosen from a (C3-C8) cycloalkyl group or a bridged tetracyclic group, said system possibly being substituted with one or more hydroxyl groups;
R3 represents:
• either a group C(O)R5 with R5 representing a (C1-C4) alkoxy group optionally substituted with a (C1-C4) alkoxy group or a group NR6R7 with R6 and R7, independently of each other, representing a hydrogen atom or a (C1-C4) alkyl, (C3-C5) cycloalkyl, (C1-C4) alkylsulfonyl or haloalkyl group,
• or a group CH₂XR8 with X representing an oxygen atom and R8 representing a hydrogen atom or a (C1-C4) alkyl group,
• or a nitrile group (CN);
p represents an integer equal to 0 or 1,
in the form of base or of addition salt with an acid or a base, and also the enantiomers and diastereoisomers thereof, including racemic mixtures thereof.

3. Compound of formula (I) according to Claim 1, **characterized in that**:
X represents a nitrogen atom and Y represents a nitrogen atom or a chain -CR4-, in which R4 represents a hydrogen atom;
A represents an aryl group, optionally substituted with one or more groups chosen from a halogen atom, (C1-C4) alkyl, (C3-C5) cycloalkyl, (C1-C4) alkoxy or haloalkoxy or represents a heteroaryl group optionally substituted with one or more groups chosen from a halogen atom or (C1-C4))alkyl;
W represents a halogen atom;
Z represents a (C1-C4) alkylene group;
B represents a group -NR4R5, in which R4 and R5 represent, independently of each other, a (C1-C4) alkyl group,
R1 and R2 represent:
• either, R1 represents a hydrogen atom and R2 represents a (C1-C4) alkyl group,
• or R1 and R2 form, together with the carbon atom to which they are attached, a (C3-C8) cycloalkyl group optionally substituted with one or more hydroxyl groups or an adamantyl group;
R3 represents:
• either a group C(O)R5 with R5 representing a (C1-C4) alkoxy group optionally substituted with a (C1-C4) alkoxy group or a group NR6R7 with R6 representing a hydrogen atom and R7 representing a hydrogen atom or a (C1-C4) alkyl, (C3-C5) cycloalkyl, (C1-C4) alkylsulfonyl or haloalkyl group,
• or a group -CH₂XR8 with X representing an oxygen atom and R8 representing a hydrogen atom or a (C1-C4) alkyl group,
p represents an integer equal to 0 or 1,
in the form of base or of addition salt with an acid or a base, and also the enantiomers and diastereoisomers thereof, including racemic mixtures thereof.

4. Compound of formula (I) according to Claim 1, **characterized in that** it is a hydrochlorid.

5. Compound of formula (I) according to Claim 1, **characterized in that** it is chosen from the following compounds:
methyl 1-{[(3,5-dichloro-2'-{4-chloro-3-[3-(dimethylamino)propoxy]phenyl}-2,3'-bipyridin-6'-yl)carbonyl]amino}cyclohexanecarboxylate;
methyl 1-({[6-{4-chloro-3-[3-(dimethylamino)propoxy]phenyl}-5-(4-hydroxy-2-methylphenyl)pyridin-2-yl]carbonyl}amino)cyclohexanecarboxylate;
methyl 1-({[6-{4-chloro-3-[3-(dimethylamino)propoxy]phenyl}-5-(5-hydroxy-2-methylphenyl)pyridin-2-yl]carbonyl}amino)cyclohexanecarboxylate;
methyl 1-({[6-{4-chloro-3-[3-(dimethylamino)propoxy]phenyl}-5-(2-methylphenyl)pyridin-2-yl]carbonyl}amino)cyclohexanecarboxylate;
methyl 1-{[(3-chloro-2'-{4-chloro-3-[3-(dimethylamino)propoxy]phenyl}-5-methyl-2,3'-bipyridin-6'-yl)carbonyl]amino}cyclohexanecarboxylate;
methyl 1-{[(6-{4-chloro-3-[3-(dimethylamino)propoxy]phenyl}-5-[5-(2-methoxyethoxy)-2-methylphenyl]pyridin-2-yl)carbonyl]amino}cyclohexanecarboxylate;
methyl 1-({[6-{4-chloro-3-[3-(dimethylamino)propoxy]phenyl}-5-(5-cyclopropyl-2-methylphenyl)pyridin-2-yl]carbonyl}amino)cyclohexanecarboxylate;
methyl 1({[6-{4-chloro-3-[3-(dimethylamino)propoxy]phenyl}-5-(5-isopropoxy-2-methylphenyl)pyridin-2-yl]carbonyl}amino)cyclohexanecarboxylate;
methyl 1-({[6-{4-chloro-3-[3-(dimethylamino)propoxy]phenyl}-5-(2-chloro-5-propoxyphenyl)pyridin-2-yl]carbonyl}amino)cyclohexanecarboxylate;
methyl 1-({[6-{4-chloro-3-[3-(dimethylamino)propoxy]phenyl}-5-(5-ethoxy-2-methylphenyl)pyridin-2-yl]carbonyl}amino)cyclohexanecarboxylate;
methyl 1-({[6-{4-chloro-3-[3-(dimethylamino)propoxy]phenyl}-5-(6-methyl-1,3-benzodioxol-5-yl)pyridin-2-yl]carbonyl}amino)cyclohexanecarboxylate;
methyl 1-{[(3-chloro-2'-{4-chloro-3-[3-(dimethylamino)propoxy]phenyl}-5-fluoro-2,3'-bipyridin-6'-yl)carbonyl]amino}cyclohexanecarboxylate;
methyl 1-{[(2'-{4-chloro-3-[3-(dimethylamino)propoxy]phenyl}-3,5-dimethyl-2,3'-bipyridin-6'-yl)carbonyl]amino}cyclohexanecarboxylate;
methyl 1-({[6-{4-chloro-3-[3-(dimethylamino)propoxy]phenyl}-5-(3-hydroxy-2-methylphenyl)pyridin-2-yl]carbonyl}amino)cyclohexanecarboxylate;
methyl 1-{[(2-{4-chloro-3-[3-(dimethylamino)propoxy]phenyl}-2'-methyl-3,3'-bipyridin-6-yl)carbonyl]amino}cyclohexanecarboxylate;
methyl 1-{[(6-{4-chloro-3-[3-(dimethylamino)propoxy]phenyl}-5-[5-(difluoromethoxy)-2-methylphenyl]pyridin-2-yl)carbonyl]amino}cyclohexanecarboxylate;
methyl 1-{[(6-{4-chloro-3-[3-(dimethylamino)propoxy]phenyl}-5-[2-(difluoromethyl)-5-methylphenyl]pyridin-2-yl)carbonyl]amino}cyclohexanecarboxylate;
methyl 1-({[6-{4-chloro-3-[3-(dimethylamino)propoxy]phenyl}-5-(3,5-dimethyl-1*H-*pyrazol-1-yl)pyridin-2-yl]carbonyl}amino)cyclohexanecarboxylate;
methyl 1-({[6-{4-chloro-3-[3-(dimethylamino)propoxy]phenyl}5-(3,5-diethyl-1*H-*pyrazol-1-yl)pyridin-2-yl]carbonyl}amino)cyclohexanecarboxylate;
methyl 1-({[6-{4-chloro-3-[3-(dimethylamino)propoxy]phenyl}-5-(2,4-dimethylphenyl)pyrazin-2-yl]carbonyl}amino)cyclohexanecarboxylate;
methyl 1-({[6-{4-chloro-3-[3-(dimethylamino)propoxy]phenyl}-5-(2-chloro-5-ethoxyphenyl)pyrazin-2-yl]carbonyl}amino)cyclohexanecarboxylate;
methyl (3*S*)-3-({[6-{4-chloro-3-[3-(dimethylamino)propoxy]phenyl}-5-(2-chloro-5-methylphenyl)pyridin-2-yl]carbonyl}amino)-4-methyl-pentanoate;
methyl (3*S*)-3-({[6-{4-chloro-3-[3-(dimethylamino)propoxy]phenyl}-5-(2,4-dimethylphenyl)pyridin-2-yl]carbonyl}amino)-4,4-dimethylpentanoate;
methyl (3*S*)-3-({[6-{4-chloro-3-[3-(dimethylamino)propoxy]phenyl}-5-(2-chloro-5-ethoxyphenyl)pyrazin-2-yl]carbonyl}amino)-4,4-dimethyl-pentanoate;
methyl (3*S*)-3-{[(3-chloro-2'-{4-chloro-3-[3-(dimethylamino)propoxy]phenyl}-5-fluoro-2,3'-bipyridin-6'-yl)carbonyl]amino}-4,4-dimethylpentanoate;
methyl 2-({[6-{4-chloro-3-[3-(dimethylamino)propoxy]phenyl}-5-(2-chlorophenyl)pyridin-2-yl]carbonyl}amino)adamantane-2-carboxylate;
2-methoxyethyl (3*S*)-3-({[6-{4-chloro-3-[3-(dimethylamino)propoxy]phenyl}-5-(2-methylphenyl)pyridin-2-yl]carbonyl}amino)-4,4-dimethyl-pentanoate;
methyl (3*S*)-3-({[6-{4-chloro-3-[3-(dimethylamino)propoxy]phenyl}-5-(2-methylphenyl)pyridin-2-yl]carbonyl}amino)-4,4-dimethyl-pentanoate;
ethyl (3*S*)-3-({[6-{4-chloro-3-[3-(dimethylamino)propoxy]phenyl}-5-(2-methylphenyl)pyridin-2-yl]carbonyl}amino)-4,4-dimethyl-pentanoate;
isopropyl (3*S*)-3-({[6-{4-chloro-3-[3-(dimethylamino)propoxy]phenyl}-5-(2-methylphenyl)pyridin-2-yl]carbonyl}amino)-4,4-dimethyl-pentanoate;
methyl (3*S*)-3-({[6-{4-chloro-3-[3-(dimethylamino)propoxy]phenyl}-5-(2-chlorophenyl)pyridin-2-yl]carbonyl}amino)-4,4-dimethyl-pentanoate;
*N*-[(1*S*)-1-(2-amino-2-oxoethyl)-2,2-dimethylpropyl]-6-{4-chloro-3-[3-(dimethylamino)propoxy]phenyl}-5-(2-methylphenyl)pyridine-2-carboxamide;
6-{4-chloro-3-[3-(dimethylamino)propoxy]phenyl}-*N-*{(1*S*)-2,2-dimethyl-1-[2-(methylamino)-2-oxoethyl]propyl}-5-(2-methylphenyl)pyridine-2-carboxamide;
6-{4-chloro-3-[3-(dimethylamino)propoxy]phenyl}-*N-*[(1*S*)-2,2-dimethyl-1-{2-[(methylsulfonyl)amino]-2-oxoethyl}propyl]-5-(2-methylphenyl)pyridine-2-carboxamide;
6-{4-chloro-3-[3-(dimethylamino)propoxy]phenyl}-*N-*[(1*S*)-1-{2-[(1,1,1-trifluoroethyl)amino]-2-oxoethyl}-2,2-dimethylpropyl]-5-(2-methylphenyl)pyridine-2-carboxamide;
6-{4-chloro-3-[3-(dimethylamino)propoxy]phenyl}-*N-*[(1*S*)-1-{2-[cyclopropyl(methyl)amino]-2-oxoethyl}-2,2-dimethylpropyl]-5-(2-methylphenyl)pyridine-2-carboxamide;
6-{4-chloro-3-[3-(dimethylamino)propoxy]phenyl}-*N-*[(1*S*)-1-(2-hydroxyethyl)-2,2-dimethylpropyl]-5-(2-methylphenyl)pyridine-2-carboxamide;
6-{4-chloro-3-[3-(dimethylamino)propoxy]phenyl}-*N-*[(1*S*)-1(2-methoxyethyl)-2,2-dimethylpropyl]-5-(2-methylphenyl)pyridine-2-carboxamide;
6-{4-chloro-3-[3-(dimethylamino)propoxy]phenyl}-*N-*[(2*S*)-1-cyano-3,3-dimethylbutan-2-yl]-5-(2-methylphenyl)pyridine-2-carboxamide;
methyl *cis*-1-{[(3,5-dichloro-2'-{4-chloro-3-[3-(dimethylamino)propoxy]phenyl}-2,3'-bipyridin-6'-yl)carbonyl]amino}-4-hydroxycyclohexanecarboxylate;
methyl *cis*-1-({[6-{4-chloro-3-[3-(dimethylamino)propoxy]phenyl}-5-(2,4-dimethylphenyl)pyridin-2-yl]carbonyl}amino)-4-hydroxycyclohexanecarboxylate;
methyl *cis*-1-({[6-{4-chloro-3-[3-(dimethylamino)propoxy]phenyl}-5-(2-methylphenyl)pyridin-2-yl]carbonyl}amino)-4-hydroxycyclohexanecarboxylate;
methyl *cis*-1-({[6-{4-chloro-3-[3-(dimethylamino)propoxy]phenyl}-5-(5-ethoxy-2-methylphenyl)pyridin-2-yl]carbonyl}amino)-4-hydroxycyclohexanecarboxylate;
methyl *cis*-1-{[(3-chloro-2'-{4-chloro-3-[3-(dimethylamino)propoxy]phenyl}-5-methyl-2,3'-bipyridin-6'-yl)carbonyl]amino}-4-hydroxycyclohexanecarboxylate.

6. Compound of formula (I) according to Claim 1, **characterized in that** it is chosen from the following compounds:
methyl 1-{[(3,5-dichloro-2'-{4-chloro-3-[3-(dimethylamino)propoxy]phenyl}-2,3'-bipyridin-6'-yl)carbonyl]amino}cyclohexanecarboxylate;
methyl 1-({[6-{4-chloro-3-[3-(dimethylamino)propoxy]phenyl}-5-(2-methylphenyl)pyridin-2-yl]carbonyl}amino)cyclohexanecarboxylate;
methyl 1-{[(3-chloro-2'-{4-chloro-3-[3-(dimethylamino)propoxy]phenyl}-5-methyl-2,3'-bipyridin-6'-yl)carbonyl]amino}cyclohexanecarboxylate;
methyl (3*S*)-3-({[6-{4-chloro-3-[3-(dimethylamino)propoxy]phenyl}-5-(2-chlorophenyl)pyridin-2-yl]carbonyl}amino)-4,4-dimethyl-pentanoate;
6-{4-chloro-3-[3-(dimethylamino)propoxy]phenyl}-*N-*[(1*S*)-2,2-dimethyl-1-{2-[(methylsulfonyl)amino]-2-oxoethyl}propyl]-5-(2-methylphenyl)pyridine-2-carboxamide;
6-{4-chloro-3-[3-(dimethylamino)propoxy]phenyl}-*N-*[(1*S*)-1-(2-hydroxyethyl)-2,2-dimethylpropyl]-5-(2-methylphenyl)pyridine-2-carboxamide;
methyl *cis*-1-({[6-{4-chloro-3-[3-(dimethylamino)propoxy]phenyl}-5-(2-methylphenyl)pyridin-2-yl]carbonyl}amino)-4-hydroxycyclohexanecarboxylate;

7. Process for preparing a compound of formula (I) according to any one of Claims 1 to 6, **characterized in that** a compound of formula (II): 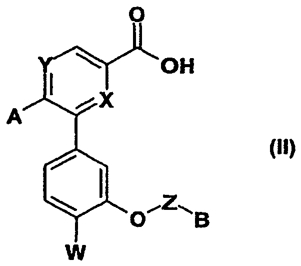 in which X, Y, Z, W, A and B are as defined in Claim 1, is reacted with a compound of formula (III): 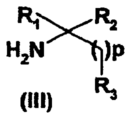 in which R1, R2 and R3 are as defined in Claim 1.

8. Process for preparing a compound of formula (I) according to any one of Claims 1 to 6, **characterized in that** a compound of formula (IV): 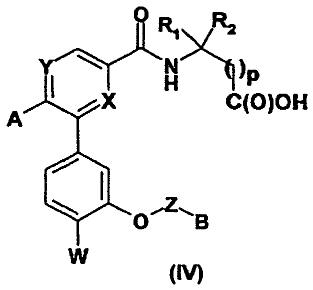 in which X, Y, Z, W, A and B are as defined in Claim 1, is reacted with a compound of formula R5H with R5 as defined in Claim 1.

9. Process for preparing a compound of formula (I) according to any one of Claims 1 to 6, **characterized in that** a compound of formula (IV): 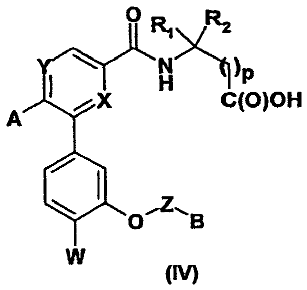 is reacted with a reducing agent.

10. Medicament, **characterized in that** it comprises a compound of formula (I) according to any one of Claims 1 to 6, or an addition salt of this compound with a pharmaceutically acceptable acid or base, or alternatively an enantiomer, a diastereoisomer or a racemic mixture of this compound.

11. Pharmaceutical composition, **characterized in that** it comprises a compound of formula (I) according to any one of Claims 1 to 6, or an addition salt of this compound with a pharmaceutically acceptable acid or base, an enantiomer, a diastereoisomer or a racemic mixture of this compound, and also at least one pharmaceutically acceptable excipient.

12. Use of a compound of formula (I) according to any one of Claims 1 to 6, for the preparation of a medicament for treating and/or preventing congestive cardiac insufficiency, cardiac ischemia, myocardial infarction, cardiac hypertrophy and fibrosis, coronary diseases and atherosclerosis, systemic and pulmonary arterial hypertension, portal hypertension, hepatic fibrosis, post-angioplasty restenosis, acute and chronic renal insufficiency of diabetic and/or hypertensive origin, diabetes, inflammation, fibroses and aneurisms, central nervous system disorders, including neurodegenerative diseases, strokes, stress, anxiety, aggressiveness, depression, schizophrenia, or sleep disorders and cancers, or respiratory diseases, and asthma.

13. Use of a compound of formula (I) according to any one of Claims 1 to 6, for the preparation of a medicament for treating and/or preventing acute and chronic renal insufficiency of diabetic origin.

14. Use of a compound of formula (I) according to any one of Claims 1 to 6, for the preparation of a medicament for treating and/or preventing diabetes.

15. Use of a compound of formula (I) according to any one of Claims 1 to 6, for the preparation of a medicament for treating and/or preventing vomiting.

## Patentansprüche

1. Verbindung entsprechend der Formel (I): 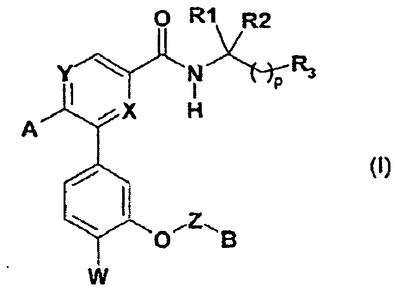 in der:
X und Y unabhängig voneinander für ein Stickstoffatom oder ein Kettenglied -CR4- stehen, wobei R4 für ein Wasserstoffatom oder eine (C1-C4)-Alkyl-oder -Alkoxygruppe steht;
A für eine Aryl- oder Heteroarylgruppe steht, wobei diese Aryl- oder Heteroarylgruppen gegebenenfalls mit einer oder mehreren Gruppen substituiert sind, die ausgewählt sind aus einem Halogenatom, einer Hydroxy-, (C1-C4)-Alkyl-, (C3-C5)-Cycloalkyl-, (C1-C4)-Alkoxygruppe, die gegebenenfalls mit einer (C1-C4)-Alkoxy-, Halogenalkyl-, Halogenalkoxygruppe, einer Nitrilgruppe substituiert sind;
W für ein Halogenatom oder eine Halogenalkylgruppe steht;
Z für eine (C1-C4)-Alkylengruppe steht, die gegebenenfalls mit einer oder mehreren Gruppen substituiert ist, die aus einem Halogenatom und (C1-C4)-Alkyl-, Hydroxy- und (C1-C4)-Alkoxygruppen ausgewählt sind;
B für eine Gruppe -NR4R5 steht, wobei R4 und R5 unabhängig voneinander für eine (C1-C4)-Alkylgruppe stehen;
R1 und R2 für Folgendes stehen:
• entweder steht R1 für ein Wasserstoffatom und R2 steht für eine (C1-C4)-Alkylgruppe,
• oder R1 und R2 bilden zusammen mit dem Kohlenstoffatom, an das sie gebunden sind, ein mono- oder polyzyklisches System, ausgewählt aus:
einer (C3-C8)-Cycloalkylgruppe, einer verbrückten bizyklischen Gruppe oder einer verbrückten tetrazyklischen Gruppe, wobei dieses System mit einer oder mehreren Hydroxygruppen substituiert sein kann;
R3 für Folgendes steht:
• entweder für eine Gruppe C(O)R5, wobei R5 für eine (C1-C4)-Alkoxygruppe steht, die gegebenenfalls mit einer (C1-C4)-Alkoxygruppe oder einer Gruppe NR6R7 substituiert ist, wobei R6 und R7 unabhängig voneinander für ein Wasserstoffatom, eine (C1-C4)-Alkyl-, (C3-C5)-Cycloalkyl-, (C1-C4)-Alkylsulfonyl, Halogenalkylgruppe stehen,
• oder für eine Gruppe -CH₂XR8, wobei:
- X für ein Sauerstoffatom steht und R8 für ein Wasserstoffatom oder eine (C1-C4)-Alkylgruppe steht,
- oder X für eine Gruppe NH steht und R8 für eine (C1-C4)-Alkylcarbonyl-, (C1-C4)-Alkylcarboxyl- oder (C1-C4)-Alkylsulfonylgruppe steht,
• oder eine Nitrilgruppe (CN);
p für eine ganze Zahl gleich 0 oder 1 steht,
im Zustand der Base oder des Additionssalzes mit einer Säure oder einer Base sowie ihre Enantiomere und Diastereoisomere, darunter ihre racemischen Gemische.

2. Verbindung der Formel (I) nach Anspruch 1, **dadurch gekennzeichnet, dass**
X und Y unabhängig voneinander für ein Stickstoffatom oder ein Kettenglied -CR4- stehen, wobei R4 für ein Wasserstoffatom steht;
A für eine Aryl- oder Heteroarylgruppe steht, wobei diese Aryl- oder Heteroarylgruppen gegebenenfalls mit einer oder mehreren Gruppen substituiert sind, die ausgewählt sind aus einem Halogenatom, einer Hydroxy-, (C1-C4)-Alkyl-, (C3-C5)-Cycloalkyl-, (C1-C4)-Alkoxygruppe, die gegebenenfalls mit einer (C1-C4)-Alkoxy-, Halogenalkyl-, Halogenalkoxygruppe substituiert sind;
W für ein Halogenatom steht;
Z für eine (C1-C4)-Alkylengruppe steht;
B für eine Gruppe -NR4R5 steht, wobei R4 und R5 unabhängig voneinander für eine (C1-C4)-Alkylgruppe stehen;
R1 und R2 für Folgendes stehen:
• entweder steht R1 für ein Wasserstoffatom und R2 steht für eine (C1-C4)-Alkylgruppe,
• oder R1 und R2 bilden zusammen mit dem Kohlenstoffatom, an das sie gebunden sind, ein mono- oder polyzyklisches System, ausgewählt aus: einer (C3-C8)-Cycloalkylgruppe und einer verbrückten tetrazyklischen Gruppe, wobei dieses System mit einer oder mehreren Hydroxygruppen substituiert sein kann; R3 für Folgendes steht:
• entweder eine Gruppe C(O)R5, wobei R5 für eine (C1-C4)-Alkoxygruppe steht, die gegebenenfalls mit einer (C1-C4)-Alkoxygruppe oder einer Gruppe NR6R7 substituiert ist, wobei R6 und R7 unabhängig voneinander für ein Wasserstoffatom, eine (C1-C4)-Alkyl-, (C3-C5)-Cycloalkyl-, (C1-C4)-Alkylsulfonyl, Halogenalkylgruppe stehen,
• oder eine Gruppe -CH₂XR8, wobei X für ein Sauerstoffatom steht und R8 für ein Wasserstoffatom oder eine (C1-C4)-Alkylgruppe steht,
• oder eine Nitrilgruppe (CN);
p für eine ganze Zahl gleich 0 oder 1 steht.
im Zustand der Base oder des Additionssalzes mit einer Säure oder einer Base sowie ihre Enantiomere und Diastereoisomere, darunter ihre racemischen Gemische.

3. Verbindung der Formel (I) nach Anspruch 1, **dadurch gekennzeichnet, dass**:
X für ein Stickstoffatom steht und Y für ein Stickstoffatom oder ein Kettenglied -CR4- steht, wobei R4 für ein Wasserstoffatom steht;
A für eine Arylgruppe steht, die gegebenenfalls mit einer oder mehreren Gruppen substituiert ist, die ausgewählt sind aus einem Halogenatom, (C1-C4)-Alkyl, (C3-C5)-Cycloalkyl, (C1-C4)-Alkoxy, Halogenalkoxy, oder für eine Heteroarylgruppe steht, die gegebenenfalls mit einer oder mehreren Gruppen substituiert ist, die ausgewählt sind aus einem Halogenatom, (C1-C4)-Alkyl;
W für ein Halogenatom steht;
Z für eine (C1-C4)-Alkylengruppe steht;
B für eine Gruppe -NR4R5 steht, wobei R4 und R5 unabhängig voneinander für eine (C1-C4)-Alkylgruppe stehen;
R1 und R2 für Folgendes stehen:
• entweder steht R1 für ein Wasserstoffatom und R2 steht für eine (C1-C4)-Alkylgruppe,
• oder R1 und R2 bilden zusammen mit dem Kohlenstoffatom, an das sie gebunden sind, eine (C3-C8)-Cycloalkylgruppe, die gegebenenfalls mit einer oder mehreren Hydroxygruppen substituiert ist, oder eine Adamantylgruppe;
R3 für Folgendes steht:
• entweder eine Gruppe C(O)R5, wobei R5 für eine (C1-C4)-Alkoxygruppe steht, die gegebenenfalls mit einer (C1-C4)-Alkoxygruppe oder einer Gruppe NR6R7 substituiert ist, wobei R6 für ein Wasserstoffatom steht und R7 für ein Wasserstoffatom, eine (C1-C4)-Alkyl-, (C3-C5)-Cycloalkyl-, (C1-C4)-Alkylsulfonyl-, Halogenalkylgruppe steht,
• oder eine Gruppe -CH₂XR8, wobei X für ein Sauerstoffatom steht und R8 für ein Wasserstoffatom oder eine (C1-C4)-Alkylgruppe steht,
p für eine ganze Zahl gleich 0 oder 1 steht,
im Zustand der Base oder des Additionssalzes mit einer Säure oder einer Base sowie ihre Enantiomere und Diastereoisomere, darunter ihre racemischen Gemische.

4. Verbindung der Formel (I) nach Anspruch 1, **dadurch gekennzeichnet, dass** sie als Hydrochlorid vorliegt.

5. Verbindung der Formel (I) nach Anspruch 1, **dadurch gekennzeichnet, dass** sie aus den folgenden Verbindungen ausgewählt ist:
Methyl-1-{[(3,5-dichlor-2'-{4-chlor-3-[3-(dimethylamino)propoxy]phenyl}-2,3'-bipyridin-6'-yl)carbonyl]amino}cyclohexancarboxylat;
Methyl-1-({[6-{4-chlor-3-[3-(dimethylamino)propoxy]phenyl}-5-(4-hydroxy-2-methylphenyl)pyridin-2-yl]carbonyl}amino)cyclohexancarboxylat;
Methyl-1-({[6-{4-chlor-3-[3-(dimethylamino)propoxy]phenyl}-5-(5-hydroxy-2-methylphenyl)pyridin-2-yl]carbonyl}amino)cyclohexancarboxylat;
Methyl-1-({[6-{4-chlor-3-[3-(dimethylamino)propoxy]phenyl}-5-(2-methylphenyl)pyridin-2-yl]carbonyl}amino)cyclohexancarboxylat;
Methyl-1-{[(3-chlor-2'-{4-chlor-3-[3-(dimethylamino)propoxy]phenyl}-5-methyl-2,3'-bipyridin-6'-yl)carbonyl]amino}cyclohexancarboxylat;
Methyl-1-{[(6-{4-chlor-3-[3-(dimethylamino)propoxy]phenyl}-5-[5-(2-methoxyethoxy)-2-methylphenyl]pyridin-2-yl)carbonyl]amino}cyclohexancarboxylat;
Methyl-1-({[6-{4-chlor-3-[3-(dimethylamino)propoxy]phenyl}-5-(5-cyclopropyl-2-methylphenyl)pyridin-2-yl]carbonyl}amino)cyclohexancarboxylat;
Methyl-1-({[6-{4-chlor-3-[3-(dimethylamino)propoxy]phenyl}-5-(5-isopropoxy-2-methylphenyl)pyridin-2-yl]carbonyl}amino)cyclohexancarboxylat;
Methyl-1-({[6-{4-chlor-3-[3-(dimethylamino)propoxy]phenyl}-5-(2-chlor-5-propoxyphenyl)pyridin-2-yl]carbonyl}amino)cyclohexancarboxylat;
Methyl-1-({[6-{4-chlor-3-[3-(dimethylamino)propoxy]phenyl}-5-(5-ethoxy-2-methylphenyl)pyridin-2-yl]carbonyl}amino)cyclohexancarboxylat;
Methyl-1-({[6-{4-chlor-3-[3-(dimethylamino)propoxy]phenyl}-5-(6-methyl-1,3-benzodioxol-5-yl)pyridin-2-yl]carbonyl}amino)cyclohexancarboxylat;
Methyl-1-{[(3-chlor-2'-{4-chlor-3-[3-(dimethylamino)propoxy]phenyl}-5-fluor-2,3'-bipyridin-6'-yl)carbonyl]amino}cyclohexancarboxylat;
Methyl-1-{[(2'-{4-chlor-3-[3-(dimethylamino)propoxy]phenyl}-3,5-dimethyl-2,3'-bipyridin-6'-yl)carbonyl]amino}cyclohexancarboxylat;
Methyl-1-({[6-{4-chlor-3-[3-(dimethylamino)propoxy]phenyl}-5-(3-hydroxy-2-methylphenyl)pyridin-2-yl]carbonyl}amino)cyclohexancarboxylat;
Methyl-1-{[(2-{4-chlor-3-[3-(dimethylamino)propoxy]phenyl}-2'-methyl-3,3'-bipyridin-6-yl)carbonyl]amino}cyclohexancarboxylat;
Methyl-1-{[(6-{4-chlor-3-[3-(dimethylamino)propoxy]phenyl}-5-[5-(difluormethoxy)-2-methylphenyl]pyridin-2-yl)carbonyl]amino}cyclohexancarboxylat;
Methyl-1-{[(6-{4-chlor-3-[3-(dimethylamino)propoxy]phenyl}-5-[2-(difluormethyl)-5-methylphenyl]pyridin-2-yl)carbonyl]amino}cyclohexancarboxylat;
Methyl-1-({[6-{4-chlor-3-[3-(dimethylamino)propoxy]phenyl}-5-(3,5-dimethyl-1*H-*pyrazol-1-yl)pyridin-2-yl]carbonyl}amino)cyclohexancarboxylat;
Methyl-1-({[6-{4-chlor-3-[3-(dimethylamino)propoxy]phenyl}-5-(3,5-diethyl-1*H-*pyrazol-1-yl)pyridin-2-yl]carbonyl}amino)cyclohexancarboxylat;
Methyl-1-({[6-{4- chlor-3-[3-(dimethylamino)propoxy]phenyl}-5-(2,4-dimethylphenyl)pyrazin-2-yl]carbonyl}amino)cyclohexancarboxylat;
Methyl-1-({[6-{4-chlor-3-[3-(dimethylamino)propoxy]phenyl}-5-(2-chlor-5-ethoxyphenyl)pyrazin-2-yl]carbonyl}amino)cyclohexancarboxylat;
Methyl-(3S)-3-({[6-{4-chlor-3-[3-(dimethylamino)propoxy]phenyl}-5-(2-chlor-5-methylphenyl)pyridin-2-yl]carbonyl}amino)-4-methylpentanoat;
Methyl-(3S)-3-({[6-{4-chlor-3-[3-(dimethylamino)propoxy]phenyl}-5-(2,4-dimethylphenyl)pyridin-2-yl]carbonyl}amino)-4,4-dimethylpentanoat;
Methyl-(3S)-3-({[6-{4-chlor-3-[3-(dimethylamino)propoxy]phenyl}-5-(2-chlor-5-ethoxyphenyl)pyrazin-2-yl]carbonyl}amino)-4,4-dimethylpentanoat;
Methyl-(3S)-3-{[(3-chlor-2'-{4-chlor-3-[3-(dimethylamino)propoxy]phenyl}-5-fluor-2,3'-bipyridin-6'-yl)carbonyl]amino}-4,4-dimethylpentanoat;
Methyl-2-({[6-{4-chlor-3-[3-(dimethylamino)propoxy]phenyl}-5-(2-chlorphenyl)pyridin-2-yl]carbonyl}amino)adamantan-2-carboxylat;
2-Methoxyethyl-(3S)-3-({[6-{4-chlor-3-[3-(dimethylamino)propoxy]phenyl}-5-(2-methylphenyl)pyridin-2-yl]carbonyl}amino)-4,4-dimethylpentanoat;
Methyl-(3S)-3-({[6-{4-chlor-3-[3-(dimethylamino)propoxy]phenyl}-5-(2-methylphenyl)pyridin-2-yl]carbonyl}amino)-4,4-dimethylpentanoat;
Ethyl-(3S)-3-({[6-{4-chlor-3-[3-(dimethylamino)propoxy]phenyl}-5-(2-methylphenyl)pyridin-2-yl]carbonyl}amino)-4,4-dimethylpentanoat;
Isopropyl-(3S)-3-({[6- {4-chlor-3-[3-(dimethylamino)propoxy]phenyl}-5-(2-methylphenyl)pyridin-2-yl]carbonyl}amino)-4,4-dimethylpentanoat;
Methyl-(3S)-3-({[6-{4-chlor-3-[3-(dimethylamino)propoxy]phenyl}-5-(2-chlorphenyl)pyridin-2-yl]carbonyl}amino)-4,4-dimethylpentanoat;
N-[(1S)-1-(2-Amino-2-oxoethyl)-2,2-dimethylpropyl]-6-{4-chlor-3-[3-(dimethylamino)propoxy]phenyl}-5-(2-methylphenyl)pyridin-2-carboxamid;
6-{4-Chlor-3-[3-(dimethylamino)propoxy]phenyl}-N-{(1S)-2,2-dimethyl-1-[2(methylamino)-2-oxoethyl]propyl}-5-(2-methylphenyl)pyridin-2-carboxamid;
6-{4-Chlor-3-[3-(dimethylamino)propoxy]phenyl}-N-[(1S)-2,2-dimethyl-1-{2-[(methylsulfonyl)amino]-2-oxoethyl}propyl]-5-(2-methylphenyl)pyridin-2-carboxamid;
6-{4-Chlor-3-[3-(dimethylamino)propoxy]phenyl}-N-[(1S)-1-{2-[(1,1,1-trifluorethyl)amino]-2-oxoethyl}-2,2-dimethylpropyl]-5-(2-methylphenyl)pyridin-2-carboxamid;
6-{4-Chlor-3-[3-(dimethylamino)propoxy]phenyl}-N-[(1S)-1-{2-[cyclopropyl(methyl)amino]-2-oxoethyl}-2,2-dimethylpropyl]-5-(2-methylphenyl)pyridin-2-carboxamid;
6-{4-Chlor-3-[3-(dimethylamino)propoxy]phenyl}-N-[(1S)-1-(2-hydroxyethyl)-2,2-dimethylpropyl]-5-(2-methylphenyl)pyridin-2-carboxamid;
6-{4-Chlor-3-[3-(dimethylamino)propoxy]phenyl}-N-[(1S)-1-(2-methoxyethyl)-2,2-dimethylpropyl]-5-(2-methylphenyl)pyridin-2-carboxamid;
6-{4-Chlor-3-[3-(dimethylamino)propoxy]phenyl}-N-[(2S)-1-cyano-3,3-dimethylbutan-2-yl]-5-(2-methylphenyl)pyridin-2-carboxamid;
Methyl-cis-1-{[(3,5-dichlor-2'-{4-chlor-3-[3-(dimethylamino)propoxy]phenyl}-2,3'-bipyridin-6'-yl)carbonyl]amino}-4-hydroxycyclohexancarboxylat;
Methyl-cis-1-({[6-{4- chlor-3-[3-(dimethylamino)propoxy]phenyl}-5-(2,4-dimethylphenyl)pyridin-2-yl]carbonyl}amino)-4-hydroxycyclohexancarboxylat;
Methyl-cis-1-({[6-{4-chlor-3-[3-(dimethylamino)propoxy]phenyl}-5-(2-methylphenyl)pyridin-2-yl]carbonyl}amino)-4-hydroxycyclohexancarboxylat;
Methyl-cis-1-({[6-{4-chlor-3-[3-(dimethylamino)propoxy]phenyl}-5-(5-ethoxy-2-methylphenyl)pyridin-2-yl]carbonyl}amino)-4-hydroxycyclohexancarboxylat;
Methyl-cis-1-{[(3-chlor-2'-{4-chlor-3-[3-(dimethylamino)propoxy]phenyl}-5-methyl-2,3'-bipyridin-6'-yl)carbonyl]amino}-4-hydroxycyclohexancarboxylat.

6. Verbindung der Formel (I) nach Anspruch 1, **dadurch gekennzeichnet, dass** sie aus den folgenden Verbindungen ausgewählt ist:
Methyl-1-{[(3,5-dichlor-2'-{4-chlor-3-[3-(dimethylamino)propoxy]phenyl}-2,3'-bipyridin-6'-yl)carbonyl]amino}cyclohexancarboxylat;
Methyl-1-({[6-{4-chlor-3-[3-(dimethylamino)propoxy]phenyl}-5-(2-methylphenyl)pyridin-2-yl]carbonyl}amino)cyclohexancarboxylat;
Methyl-1-{[(3-chlor-2'-{4-chlor-3-[3-(dimethylamino)propoxy]phenyl}-5-methyl-2,3'-bipyridin-6'-yl)carbonyl]amino}cyclohexancarboxylat;
Methyl-(3S)-3-({[6-{4-chlor-3-[3-(dimethylamino)propoxy]phenyl}-5-(2-chlorphenyl)pyridin-2-yl]carbonyl}amino)-4,4-dimethylpentanoat;
6-{4-Chlor-3-[3-(dimethylamino)propoxy]phenyl}-N-[(1S)-2,2-dimethyl-1-{2-[(methylsulfonyl)amino]-2-oxoethyl}propyl]-5-(2-methylphenyl)pyridin-2-carboxamid;
6-{4-Chlor-3-[3-(dimethylamino)propoxy]phenyl}-N-[(1S)-1-(2-hydroxyethyl)-2,2-dimethylpropyl]-5-(2-methylphenyl)pyridin-2-carboxamid;
Methyl-cis-1-({[6-{4-chlor-3-[3-(dimethylamino)propoxy]phenyl}-5-(2-methylphenyl)pyridin-2-yl]carbonyl}amino)-4-hydroxycyclohexancarboxylat;

7. Verfahren zur Herstellung einer Verbindung der Formel (I) nach einem der Ansprüche 1 bis 6, **dadurch gekennzeichnet, dass** man eine Verbindung der Formel (II): 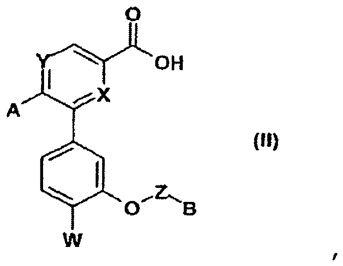 in der X, Y, Z, W, A und B wie im Anspruch 1 definiert sind, mit einer Verbindung der Formel (III): 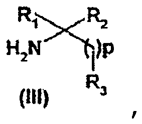 in der R1, R2 und R3 wie im Anspruch 1 definiert sind, umsetzt.

8. Verfahren zur Herstellung einer Verbindung der Formel (I) nach einem der Ansprüche 1 bis 6, **dadurch gekennzeichnet, dass** man eine Verbindung der Formel (IV) : 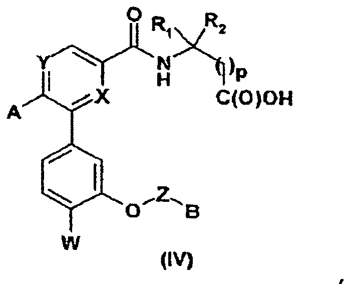 in der X, Y, Z, W, A und B wie im Anspruch 1 definiert sind, mit einer Verbindung der Formel R5H, wobei R5 wie im Anspruch 1 definiert ist, umsetzt.

9. Verfahren zur Herstellung einer Verbindung der Formel (I) nach einem der Ansprüche 1 bis 6, **dadurch gekennzeichnet, dass** man eine Verbindung der Formel (IV) : 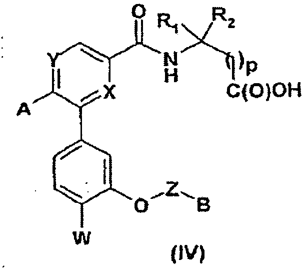 mit einem Reduktionsmittel umsetzt.

10. Arzneimittel, **dadurch gekennzeichnet, dass** es eine Verbindung der Formel (I) nach einem der Ansprüche 1 bis 6 oder ein Additionssalz dieser Verbindung mit einer pharmazeutisch annehmbaren Säure oder Base oder auch ein Enantiomer, ein Diastereoisomer oder ein racemisches Gemisch dieser Verbindung umfasst.

11. Pharmazeutische Zusammensetzung, **dadurch gekennzeichnet, dass** sie eine Verbindung der Formel (I) nach einem der Ansprüche 1 bis 6 oder ein Additionssalz dieser Verbindung mit einer pharmazeutisch annehmbaren Säure oder Base, ein Enantiomer, ein Diastereoisomer oder ein racemisches Gemisch dieser Verbindung sowie mindestens einen pharmazeutisch annehmbaren Excipienten umfasst.

12. Verwendung einer Verbindung der Formel (I) nach einem der Ansprüche 1 bis 6 zur Herstellung eines Arzneimittels zur Behandlung und/oder Prävention von kongestiver Herzinsuffizienz, Herzischämie, Myokardinfarkt, Herzhypertrophie und Herzfibrose, Koronarkrankheiten und Atherosklerose, von arteriellem systemischem und pulmonalem Bluthochdruck, Pfortaderbluthochdruck, Leberfibrose, von Restenose nach Angioplastie, akuter und chronischer Niereninsuffizienz mit Ursprung in Diabetes und/oder Hypertonie, Diabetes, Entzündung, Fibrosen und Aneurismen, Störungen des zentralen Nervensystems, einschließlich neurodegenerativer Krankheiten, Schlaganfällen, Stress, Angst, Aggressivität, Depression, Schizophrenie oder auch Schlafstörungen und Krebserkrankungen oder Atemwegserkrankungen, wie Asthma.

13. Verwendung einer Verbindung der Formel (I) nach einem der Ansprüche 1 bis 6 zur Herstellung eines Arzneimittels zur Behandlung und/oder Prävention von akuter und chronischer Niereninsuffizienz mit Ursprung in Diabetes.

14. Verwendung einer Verbindung der Formel (I) nach einem der Ansprüche 1 bis 6 zur Herstellung eines Arzneimittels zur Behandlung und/oder Prävention von Diabetes.

15. Verwendung einer Verbindung der Formel (I) nach einem der Ansprüche 1 bis 6 zur Herstellung eines Arzneimittels zur Behandlung und/oder Prävention von Erbrechen.
